# EUROPÄISCHE PATENTSCHRIFT

(11) **EP 4 289 407 B1**
(45) Veröffentlichungstag und Bekanntmachung des Hinweises auf die Patenterteilung: **29.07.2026**
(21) Anmeldenummer: 23176681.7
(22) Anmeldetag: 01.06.2023
(51) Int. Cl.: A61F 13/49, A61F 13/56

(54) **ANORDNUNG VON INKONTINENZWEGWERFWINDELN**
ARRANGEMENT OF DISPOSABLE INCONTINENCE DIAPERS
ENSEMBLE DE COUCHES JETABLES POUR INCONTINENCE

(30) Priorität: 09.06.2022 DE 102022114510
(43) Veröffentlichungstag der Anmeldung: 13.12.2023
(73) Patentinhaber: Paul Hartmann AG, 89522 Heidenheim (DE)
(72) Erfinder: Krämer, Tobias, 91443 Scheinfeld (DE)
(74) Vertreter: Paul Hartmann AG Patents & Licensing

(56) Entgegenhaltungen:
- EP-B1- 2 934 406
- WO-A1-2020/069967
- US-A1- 2008 275 415
- US-A1- 2016 354 262

## Beschreibung

Die vorliegende Erfindung betrifft eine Anordnung von Inkontinenzwegwerfwindeln des offenen Typs für die Aufnahme von Körperausscheidungen für die bevorzugte Verwendung durch Erwachsene.

Gattungsgemäße Inkontinenzwegwerfwindeln sind bekannt und weisen häufig einen Hauptteil (Chassis), bestehend aus einem Vorderbereich, einem Rückenbereich und einem in Längsrichtung der Windel dazwischen liegenden zwischen den Beinen eines Benutzers zu liegen kommenden Schrittbereich auf, wobei der Hauptteil meist bereits einen Flüssigkeiten absorbierenden Absorptionskörper umfasst, und mit an den Rückenbereich und/oder den Vorderbereich beidseitig angefügten, voneinander separaten und vorzugsweise mit Verschlussmitteln versehenen Seitenabschnitten, welche sich in Querrichtung der Windel über seitliche Längsränder des Hauptteils hinaus erstrecken und den Vorderbereich und den Rückenbereich im angelegten Zustand der Windel miteinander verbinden.

Dieser offene Windel-Typ wird, im Gegensatz zu "pant-type", üblicherweise derart an den Körper angelegt, dass die an den Rückenbereich angefügten Seitenabschnitte der Windel in je nach anatomischen Gegebenheiten unterschiedlich starker Überlappung mit einer Außenseite des Vorderbereichs der Windel oder auch mit den an den Vorderbereich der Windel angefügten Seitenabschnitten gebracht und dort lösbar insbesondere mittels klebenden oder mechanischen Verschlussmitteln angeheftet werden. Eine derartige Windel ist beispielsweise aus WO2005/102241A1 bekannt.

Um der unterschiedlichen Körpergröße von verschiedenen Benutzern Rechnung zu tragen sind beispielsweise aus WO2020/069953A1 Anordnungen von Inkontinenzwegwerfwindeln unterschiedlicher Größe bekannt, die sich bezüglich Chassislänge und Spannweite in Hüftumfangsrichtung unterscheiden. Für Benutzer mit geringerer Körpergröße werden also kleiner dimensionierte, ansonsten jedoch im Wesentlichen baugleiche Windeln bereitgestellt. Es hat sich jedoch gezeigt, dass insbesondere bei Benutzern hoher Bewegungsaktivität und eher geringer Körpergröße oder eher schmaler Statur, wie beispielsweise Jugendliche oder zierliche Erwachsene, ein Bedarf an verbesserten Inkontinenzwegwerfwindeln besteht, die den anatomischen Gegebenheiten und der Anwendungssituation stärker Rechnung tragen.

Weiterer Stand der Technik ist in US2016/354262 offenbart.

Der vorliegenden Erfindung liegt daher die Aufgabe zu Grunde, Inkontinenzwegwerfwindeln mit guter Passform und hoher Auslaufsicherheit bereitzustellen, die unterschiedlichen Benutzergruppen Rechnung tragen.

Diese Aufgabe wird gelöst durch eine Anordnung gemäß Anspruch 1.

Vorzugsweise beträgt die Chassislänge der von der Anordnung umfassten Inkontinenzwegwerfwindeln 500 mm -1100 mm, insbesondere 550 mm -1050 mm, vorzugsweise 580 mm -1000 mm.

An dieser Stelle sei ausgeführt, dass in der vorliegenden Anmeldung jegliche Bemessungsangaben hinsichtlich Quererstreckung, Längserstreckung von Abschnitten oder von an sich beliebigen Komponenten der Inkontinenzwegwerfwindel in eben ausgebreitetem Zustand der den Artikel bildenden Flachmaterialien bestimmt werden, so dass der betreffende Artikel in die in den Figuren dargestellte ebene Konfiguration gebracht werden kann, sofern kein anderer Hinweis auf einen davon abweichenden Zustand angeführt ist. Wenn der Artikel beispielsweise durch fadenförmige Elastifizierungsmittel im sogenannten "Stretchbond-Verfahren" elastifiziert wurde, so werden die Flächenmaterialien, wie in den Figuren angedeutet, derart ausgedehnt betrachtet, wie sie herstellerseitig als Flachmaterialien zugeführt werden oder nachträglich bis zu ihrer natürlichen Ausgangserstreckung ohne Elastifizierungsmittel ausgebreitet und auf eine ebene Fläche aufgelegt werden können. In dieser ebenen Fläche werden dann die Quererstreckungen, Längserstreckungen oder Abmessungen ermittelt. Dieser Zustand ergibt sich bei im Wesentlichen undehnbaren Flachmaterialien auf Vliesbasis oder Folienbasis oder Vlies-Folienverbundbasis auf natürliche Weise.

Das Chassis, die Seitenabschnitte und die Windelohren der von der Anordnung umfassten Inkontinenzwegwerfwindeln sind vorzugsweise im Wesentlichen undehnbar, insbesondere umfassend im Wesentlichen undehnbare Flachmaterialien, ausgebildet, um einem Verrutschen der flüssigkeitsabsorbierenden Bereiche, mithin des Absorptionskörpers, im Gebrauch entgegenzuwirken.

Die im Wesentlichen undehnbaren Flachmaterialien umfassen oder bestehen aus vorzugsweise einem Vliesmaterial, wie Spinnvlies, Meltblownvlies, Kardenvlies, Spunlacevlies oder Through Air bonded Kardenvlies oder Kombinationen hiervon. Bevorzugt sind Spinnvliesmaterialien (Spunbondvliesstoffe) und/oder Meltblownvliesmaterialien, insbesondere Laminate aus Spunbond- (S) und Meltblown- (M) Vliesstoffen oder Vliesschichten wie SMS-, oder SSMS- oder SMMS-, oder SSMMS- oder SSMMSS-Laminate.

Es hat sich weiter als vorteilhaft erwiesen, wenn das Vliesmaterial zumindest eine Rezepturkomponente auf Basis eines thermoplastischen Polymers enthält, wie Polyethylen PE, Polypropylen PP oder Polyethylenterephthalat PET oder Mischungen davon. Vliesmaterialien umfassend Rayon, Cellulose, Polyamid PA und Mischungen davon sind ebenfalls denkbar.

Die Chassislänge einer Inkontinenzwegwerfwindel ist zu verstehen als maximale Längserstreckung der Inkontinenzwegwerfwindel gemessen in mm von einem hinteren Querrand des Chassis der Inkontinenzwegwerfwindel bis zu einem vorderen Querrand des Chassis der Inkontinenzwegwerfwindel.

Die erfindungsgemäße Anordnung umfasst also Inkontinenzwegwerfwindeln unterschiedlicher Längserstreckung und damit unterschiedlicher Windelgröße, um eine jeweils passende Größe für Benutzer unterschiedlicher Körpergröße oder Statur bereitzustellen.

Die dritte Inkontinenzwegwerfwindel weist eine geringere Chassislänge auf als die erste und die zweite Inkontinenzwegwerfwindel, und ist daher für Benutzer mit geringeren Körpermaßen vorgesehen. Dabei unterscheidet sich die dritte Inkontinenzwegwerfwindel von der ersten und der zweiten Inkontinenzwegwerfwindel in weiteren Merkmalen, um den anatomischen Besonderheiten der Benutzer stärker Rechnung zu tragen.

Es wurde nämlich festgestellt, dass Benutzer geringerer Körpergröße häufiger einen zierlicheren Körperbau oder eine schmalere Statur aufweisen. Beispielsweise kann es sich dabei um Jugendliche handeln, die noch im Wachstum begriffen sind oder um schlanke Erwachsene. Dabei erweist sich die konturierte Beinöffnung der dritten Inkontinenzwegwerfwindel als vorteilhaft, weil sie sich besser an eine schlankere Körperform anschmiegt und/oder Oberschenkel mit geringerem Umfang sicherer umschließt. Dadurch wird einerseits die Passform und der Tragekomfort kleiner Inkontinenzwegwerfwindeln verbessert, andererseits auch insbesondere bei bewegungsaktiven Benutzern die Auslaufsicherheit und das Zutrauen des Benutzers zum Produkt erhöht.

Typischerweise sind die angefügten Seitenabschnitte derartiger Inkontinenzwegwerfwindeln rechteckig oder trapezförmig ausgebildet, da dies aus der Fertigungssicht die einfachste, effizienteste und kostengünstigste Form mit vergleichsweise geringem Materialabfall ist.

Die Beinöffnung der ersten und der zweiten Inkontinenzwegwerfwindel ist gebildet und begrenzt durch jeweils einen seitlichen freien Längsrand des Schrittbereichs und einen an diesen seitlichen freien Längsrand angrenzenden inneren, also dem Schrittbereich der Inkontinenzwegwerfwindel zugewandten, Querrand des jeweiligen hinteren Seitenabschnitts und einen an diesen seitlichen freien Längsrand angrenzenden inneren, also dem Schrittbereich der Inkontinenzwegwerfwindel zugewandten, Querrand des jeweiligen vorderen Seitenabschnitts.

Der seitliche freie Längsrand des Schrittbereichs bildet mit dem inneren Querrand des hinteren Seitenabschnitts einen im Wesentlichen rechten Winkel, nämlich einen Winkel α von 85° bis 100°, insbesondere einen Winkel α von genau 90°. Der seitliche freie Längsrand des Schrittbereichs bildet mit dem inneren Querrand des vorderen Seitenabschnitts einen im Wesentlichen rechten Winkel, nämlich einen Winkel β von 85° bis 100°, insbesondere einen Winkel β von genau 90°. Der Scheitelpunkt ist zu verorten, wo der seitliche freie Längsrand des Schrittbereichs auf die innere Querkante des jeweiligen hinteren oder vorderen Seitenabschnitts trifft, dabei in Querrichtung außerhalb eines Überlappungsbereiches von Hauptteil und jeweiligem hinteren oder vorderen Seitenabschnitt. Der Winkel α und der Winkel β können identisch oder verschieden sein. Sowohl der seitliche freie Längsrand des Schrittbereichs als auch der innere Querrand des hinteren Seitenabschnitts als auch der innere Querrand des vorderen Seitenabschnitts sind bevorzugt jeweils geradlinig ausgebildet, können jedoch alternativ auch einen kurvenförmigen, gebogenen, geschwungenen, konvexen und/oder konkaven Verlauf aufweisen. Bei nicht geradlinigem Verlauf des seitlichen freien Längsrands wird zur Bestimmung des Winkels α und/oder des Winkels β statt des seitlichen freien Längsrands eine vom Scheitelpunkt des jeweiligen Winkels α oder Winkels β ausgehende und zur Längsrichtung der Inkontinenzwegwerfwindel parallele imaginäre gerade Linie herangezogen. Bei nicht geradlinigem Verlauf des inneren Querrands des hinteren Seitenabschnitts oder des inneren Querrands des vorderen Seitenabschnitts wird zur Bestimmung des jeweiligen Winkels α oder Winkels β statt des jeweiligen inneren Querrands des hinteren oder vorderen Seitenabschnitts eine vom Scheitelpunkt des jeweiligen Winkels α oder Winkels β ausgehende und an einem in Querrichtung der Inkontinenzwegwerfwindel äußersten und dabei dem Schrittbereich der Inkontinenzwegwerfwindel nächstgelegenen Punkt des jeweiligen hinteren oder vorderen Seitenabschnitts endende imaginäre gerade Linie herangezogen.

Jeder der hinteren Seitenabschnitte der ersten und der zweiten Inkontinenzwegwerfwindel weist jeweils zwei Verschlussmittel auf, die in Längsrichtung voneinander beabstandet sind. Das ermöglicht es einerseits, auf materialsparende Weise eine effektive Verschlussfunktion über einen größeren Bereich in Längsrichtung des jeweiligen Seitenabschnitts bereitzustellen. Andererseits können durch die Beabstandung in Längsrichtung ein jeweiliges inneres und äußeres Verschlussmittel eines Seitenabschnitts relativ frei und unabhängig voneinander, mithin auch nicht parallel zueinander, beim Anlegen der Inkontinenzwegwerfwindel platziert werden, so dass die Passform und die Auslaufsicherheit verbessert werden kann.

Die dritte Inkontinenzwegwerfwindel weist hintere und vordere Windelohren auf, die jeweils in struktureller Einheit mit dem Hauptteil ausgebildet sind. Die Formulierung "in struktureller Einheit mit dem Hauptteil" ist im Rahmen dieser Erfindung so zu verstehen, dass die Windelohren nicht aus separaten Materialabschnitten ausgebildet und an den Hauptteil angefügt sind, sondern mit dem Hauptteil gemeinsam aus einem einzigen Materialabschnitt ausgebildet, beispielsweise mit dem Hauptteil zusammen in einem Stück aus einem Materialabschnitt ausgeschnitten, sind. Vorteile hierbei sind, dass in der Fertigung von Inkontinenzwegwerfwindeln kleiner Größe Prozessschritte zur separaten Ausbildung der Windelohren und des Hauptteils sowie des Aneinanderfügens dieser Komponenten entfallen, die aufgrund der geringen Größe der Materialabschnitte schwierig sein können. Außerdem kann eine Fügestelle zwischen Seitenabschnitt und Hauptteil aufgrund der gerade bei kleinen Größen und insbesondere bei bewegungsaktiven Benutzern verstärkt auftretenden hohen Zugkräfte als Schwachstelle wahrgenommen werden, wohingegen die strukturelle Einheit von Hauptteil und Windelohren ein höheres Zutrauen des Benutzers in die Robustheit der Inkontinenzwegwerfwindel und damit ein besseres Sicherheitsgefühl bewirken kann. Der Hauptteil und/oder die hinteren Windelohren und/oder die vorderen Windelohren können zusätzliche Materiallagen aufweisen, welche sich nicht über die jeweiligen anderen Windelteile, nämlich Hauptteil oder hintere Windelohren oder vordere Windelohren, hinweg erstrecken. Beispielsweise können der Hauptteil und die hinteren Windelohren und die vorderen Windelohren aus einem einzigen Vliesmaterialabschnitt ausgebildet sein und der Hauptteil kann eine zusätzliche Lage eines im Wesentlichen wasserundurchlässigen Materials, beispielsweise eines Folienmaterials, umfassen, welches sich nicht über die hinteren und/oder die vorderen Windelohren hinweg erstreckt.

Zum Anlegen weist die dritte Inkontinenzwegwerfwindel beidseits jeweils nur ein Verschlussmittel auf, wodurch die Windel schneller angelegt werden kann. Aufgrund der geringeren Chassislänge der dritten Inkontinenzwegwerfwindel und der dadurch geringeren möglichen Beabstandung bei zwei oder mehr Verschlussmitteln kann darüber hinaus die Gefahr bestehen, dass die einzelnen Verschlussmittel sich beim Anlegen leichter ineinander verschlingen oder verfangen, insbesondere wenn die Inkontinenzwegwerfwindel durch eine Pflegekraft bei einem bewegungsaktiven und/oder unruhigen Benutzer angelegt wird. Daher bietet ein einziges Verschlussmittel hier einen Handhabungsvorteil.

Weiterhin weist die dritte Inkontinenzwegwerfwindel eine konturierte Beinöffnung auf. Der Begriff "konturierte Beinöffnung" ist im Rahmen der Erfindung so zu verstehen, dass ein innerer Querrand des hinteren Windelohrs, ein seitlicher freier Längsrand des Schrittbereichs und ein innerer Querrand des vorderen Windelohrs gemeinsam eine kontinuierliche, stetige, zumindest bereichsweise kurvenförmige Materialaussparung im Schrittbereich begrenzen. Die dritte Inkontinenzwegwerfwindel erhält hierdurch eine sanduhrförmige Gestalt. Eine beim Anlegen oder bei erhöhter Bewegungsaktivität des Benutzers in der Gebrauchssituation mittels der Verschlussmittel auf die hinteren Windelohren ausgeübte Zugkraft wird hierbei über eine größere Fläche und hin zu einem mittleren Bereich des Schrittbereichs verteilt, wodurch die Reißfestigkeit der Inkontinenzwegwerfwindel, insbesondere die Ausreißfestigkeit der hinteren Windelohren, verbessert sein kann.

Dabei passt sich der Schrittbereich der dritten Inkontinenzwegwerfwindel der Anatomie des Benutzers besser an und die Inkontinenzwegwerfwindel kann insbesondere einen Oberschenkel mit geringerem Umfang sicherer umschließen. Dadurch wird einerseits die Passform und der Tragekomfort kleiner Inkontinenzwegwerfwindeln verbessert, andererseits auch insbesondere bei bewegungsaktiven Benutzern die Auslaufsicherheit und das Zutrauen des Benutzers zum Produkt erhöht.

Die Vorteile der dritten Inkontinenzwegwerfwindel für Benutzer geringerer Körpergröße oder schmalerer Statur ergeben sich insbesondere durch die Kombination der sie von der ersten und zweiten Inkontinenzwegwerfwindel unterscheidenden Merkmale. Beispielsweise kann beim Anlegen der ersten oder zweiten Inkontinenzwegwerfwindel durch eine schräge Anordnung eines dem Schrittbereich nächstgelegenen Verschlussmittels die Passform an einen jeweiligen Benutzer und damit die Auslaufsicherheit optimiert werden. Aufgrund der geringeren Größe wäre jedoch bei einer Inkontinenzwegwerfwindel, die die Merkmale der ersten oder zweiten Inkontinenzwegwerfwindel aufweist und dabei lediglich mit geringeren Abmessungen ausgebildet ist, nicht im gleichen Maße möglich, insbesondere bei bewegungsaktiven Benutzern. Die Kombination des einen Verschlussmittels mit der konturierten Beinöffnung hingegen ermöglicht ein schnelleres Anlegen und sicheres Verschließen der dritten Inkontinenzwegwerfwindel bei reduzierter Gefahr des Auslaufens. Insgesamt erlaubt die Anordnung umfassend die erste, zweite und dritte Inkontinenzwegwerfwindel auf vorteilhafte Weise, bei sehr ähnlicher Handhabung der jeweiligen Inkontinenzwegwerfwindel verschiedenen Benutzern mit unterschiedlicher Körpergröße oder Statur eine an die jeweiligen Bedürfnisse angepasste Inkontinenzwegwerfwindel mit gewohnt guter Auslaufsicherheit und Passform bereitzustellen.

Eine Anordnung umfassend eine erste, zweite und dritte Inkontinenzwegwerfwindel wird verstanden als zumindest jeweils einem Vertreter davon und schließt eine Mehrzahl der jeweiligen ersten und/oder zweiten und/oder dritten Inkontinenzwegwerfwindel ein oder solche enthaltenden Packungen und Verpackungseinheiten.

In einer bevorzugten Ausführungsform umfasst die Anordnung eine vierte Inkontinenzwegwerfwindel mit einer Chassislänge CL4, einer Längsrichtung, einer Querrichtung orthogonal zu der Längsrichtung, einem zumindest bereichsweise flüssigkeitsdurchlässigen Topsheet, einem im Wesentlichen flüssigkeitsundurchlässigen Backsheet und einem zwischen dem Topsheet und dem Backsheet angeordneten Absorptionskörper,
- wobei gilt CL3>CL4,
- wobei die vierte Inkontinenzwegwerfwindel einen Hauptteil umfassend einen Vorderbereich und einen Rückenbereich und einen in der Längsrichtung dazwischen angeordneten, zwischen den Beinen eines Benutzers zu liegen kommenden Schrittbereich umfasst,
- wobei die vierte Inkontinenzwegwerfwindel im Rückenbereich beidseits in Querrichtung sich hinauserstreckende hintere Windelohren und im Vorderbereich beidseits in Querrichtung sich hinauserstreckende vordere Windelohren aufweist, wobei die hinteren Windelohren und die vorderen Windelohren in struktureller Einheit mit dem Hauptteil ausgebildet sind,
- wobei jedes der beiden hinteren Windelohren jeweils ein einziges Verschlussmittel aufweist, so dass die hinteren Windelohren zum Anlegen und Schließen der vierten Inkontinenzwegwerfwindel an einen Benutzer jeweils entlang einer Umfangsrichtung um den Körper des Benutzers herumlegbar und in überlappende Anordnung mit einer Außenseite des Vorderbereiches und/oder einer Außenseite der vorderen Windelohren bringbar sind, an der sie über das jeweilige Verschlussmittel jeweils lösbar anhaftbar sind,
- und wobei die vierte Inkontinenzwegwerfwindel im Schrittbereich beidseits eine konturierte Beinöffnung aufweist.

Die bevorzugte Anordnung umfassend die vierte Inkontinenzwegwerfwindel unterstützt weiter und auf vorteilhafte Weise die Bereitstellung von Inkontinenzwegwerfwindeln mit ähnlicher Handhabung und mit gewohnt guter Auslaufsicherheit und Passform für Benutzer mit unterschiedlicher Körpergröße oder Statur.

Bekannte Inkontinenzwegwerfwindeln sind im Stand der Technik häufig mit einer Kennzeichnung versehen, die dem Benutzer Aufschluss über eine jeweilige Windelgröße geben. Beispielsweise kann die erste Inkontinenzwegwerfwindel als große Größe (im Englischen auch "large" oder "L"), die zweite Inkontinenzwegwerfwindel als mittlelgroße Größe ("medium" oder "M"), die dritte Inkontinenzwegwerfwindel als kleine Größe ("small" oder "S") und die optionale vierte Inkontinenzwegwerfwindel als extra kleine Größe ("extra small" oder "XL") Größe bezeichnet sein.

Es ist denkbar, dass die Anordnung in einer weiteren Ausführungsform eine fünfte Inkontinenzwegwerfwindel mit einer Chassislänge CL5 umfasst, wobei gilt CL5>CL4 und wobei die fünfte Inkontinenzwegwerfwindel zumindest die oben mit Bezug zur ersten Inkontinenzwegwerfwindel beschriebenen Merkmale aufweist. Die fünfte Inkontinenzwegwerfwindel kann beispielsweise als extra große Größe ("extra large" oder "XL") bezeichnet sein.

Weiterhin ist denkbar, dass die Anordnung Inkontinenzwegwerfwindeln unterschiedlicher Absorptionskapazität umfasst, dass also Inkontinenzwegwerfwindeln gleicher Größe oder Inkontinenzwegwerfwindeln unterschiedlicher Größe jeweils eine unterschiedliche Absorptionskapazität aufweisen. Die jeweilige Absorptionskapazität ist hierbei in einer dem Fachmann an sich bekannter Weise nach ISO 11948-1 (1996) zu messen.

In einer bevorzugten Ausführungsform weist mindestens eines der Verschlussmittel der ersten und/oder der zweiten Inkontinenzwegwerfwindel eine erste Verschlussmittellänge VL1 in der Längsrichtung der Inkontinenzwegwerfwindel auf und mindestens eines der Verschlussmittel der dritten und/oder der vierten Inkontinenzwegwerfwindel weist eine zweite Verschlussmittellänge VL2 in der Längsrichtung der Inkontinenzwegwerfwindel auf, und es gilt VL1<VL2. Dadurch kann die Verschlusssicherheit der dritten und/oder der optionalen vierten Inkontinenzwegwerfwindel trotz der geringeren Anzahl an Verschlussmitteln verbessert werden, so dass insbesondere bei bewegungsaktiven Benutzern die Inkontinenzwegwerfwindel ein sicherer Halt unterstützt wird.

Ein bevorzugtes Verhältnis von VL2 zu VL1 weist einen Wert von 1,2-3,0, insbesondere 1,3-2,7, weiter insbesondere 1,5-2,5, weiter insbesondere 1,8-2,2, weiter insbesondere von 2,0 auf. Es wurde nämlich gefunden, dass ein solches Verhältnis von VL2 zu VL1 den sicheren Halt der dritten und/oder der vierten Inkontinenzwegwerfwindel mit nur einem einzigen Verschlussmittel an jedem der hinteren Windelohren ausreichend unterstützt, gleichsam den auftretenden Zugkräften im Gebrauch ausreichend standhalten kann, und gleichzeitig in ähnlicher und gewohnter Weise zu handhaben ist wie die Verschlussmittel der ersten und/oder zweiten Inkontinenzwegwerfwindel.

In einem bevorzugten Ausführungsbeispiel weist die Beinöffnung der ersten Inkontinenzwegwerfwindel eine erste Beinöffnungslänge BL1 auf, und die Beinöffnung der zweiten Inkontinenzwegwerfwindel weist eine zweite Beinöffnungslänge BL2 auf, und die konturierte Beinöffnung der dritten Inkontinenzwegwerfwindel weist eine dritte Beinöffnungslänge BL3 auf, wobei insbesondere gilt BL1>BL2 und BL2>BL3.

Weiter bevorzugt weist die konturierte Beinöffnung der vierten Inkontinenzwegwerfwindel eine vierte Beinöffnungslänge BL4 auf, wobei insbesondere gilt BL3>BL4.

Hierdurch kann für verschiedene Benutzer mit jeweiligen unterschiedlichen Körpermaßen oder unterschiedlicher Statur, insbesondere mit unterschiedlichen Oberschenkelmaßen, eine Inkontinenzwegwerfwindel mit verbesserter Passform und Auslaufsicherheit bereitgestellt werden.

Die jeweilige Beinöffnungslänge der ersten, zweiten, dritten und der optionalen vierten Inkontinenzwegwerfwindel entspricht einem maximalen Abstand des inneren Querrands des jeweiligen hinteren Seitenabschnitts oder des hinteren Windelohres zum inneren Querrand des jeweiligen vorderen Seitenabschnitts oder des vorderen Windelohres, gemessen in Längsrichtung der Inkontinenzwegwerfwindel.

In einem bevorzugten Ausführungsbeispiel weist ein Verhältnis der Chassislänge zur Beinöffnungslänge der ersten Inkontinenzwegwerfwindel CL1/BL1 und der zweiten Inkontinenzwegwerfwindel CL2/BL2 und der dritten Inkontinenzwegwerfwindel CL3/BL3 jeweils einen Wert von 1,6-2,4, insbesondere von 1,7-2,3, weiter insbesondere von 1,8-2,2, weiter insbesondere 1,9-2,2 auf.

Weiter bevorzugt weist ein Verhältnis der Chassislänge zur Beinöffnungslänge der vierten Inkontinenzwegwerfwindel CL4/BL4 einen Wert von 1,6-2,4, insbesondere von 1,7-2,3, weiter insbesondere von 1,8-2,2, weiter insbesondere 1,9-2,2 auf.

Dadurch ergibt sich auf vorteilhafte Weise eine ähnliche Handhabung beim Anlegen an einen jeweiligen Benutzer und eine ähnliche Passform bei den von der Anordnung umfassten Inkontinenzwegwerfwindeln. Außerdem können hierdurch auf vorteilhafte Weise Inkontinenzwegwerfwindeln mit im Wesentlichen gleicher Auslaufsicherheit bereitgestellt werden, so dass das Zutrauen der Benutzer in die von der Anordnung umfassten Inkontinenzwegwerfwindeln unterstützt wird.

In einer bevorzugten Ausführungsform weist die Beinöffnungslänge der Beinöffnung der ersten Inkontinenzwegwerfwindel BL1 und der zweiten Inkontinenzwegwerfwindel BL2 einen Wert von 380-490 mm, insbesondere 390-480 mm, weiter insbesondere 400-470 mm, weiter insbesondere 410-460 mm auf. Weiter bevorzugt weist die Beinöffnungslänge der konturierten Beinöffnung der dritten Inkontinenzwegwerfwindel BL3 einen Wert von 280-370 mm, insbesondere von 290-360 mm, weiter insbesondere von 300-350 mm auf. Weiter bevorzugt weist die Beinöffnungslänge der konturierten Beinöffnung der vierten Inkontinenzwegwerfwindel BL4 einen Wert von 280-370 mm, insbesondere von 290-360 mm, weiter insbesondere von 300-350 mm auf.

Es wurde nämlich gefunden, dass eine Anordnung von Inkontinenzwegwerfwindeln mit den vorgenannten Beinöffnungslängen erlaubt, auf vorteilhafte Weise geeignete Inkontinenzwegwerfwindeln für ein großes Spektrum an unterschiedlichen Benutzern mit unterschiedlichen Körpermaßen oder Statur bereitzustellen.

In einem bevorzugten Ausführungsbeispiel ist eine minimale Schrittbereichsbreite der ersten Inkontinenzwegwerfwindel SB1 und eine minimale Schrittbereichsbreite der zweiten Inkontinenzwegwerfwindel SB2 jeweils größer als eine minimale Schrittbereichsbreite der dritten Inkontinenzwegwerfwindel SB3. Weiter bevorzugt ist die minimale Schrittbereichsbreite der ersten Inkontinenzwegwerfwindel SB1 und die minimale Schrittbereichsbreite der zweiten Inkontinenzwegwerfwindel SB2 jeweils größer als eine minimale Schrittbereichsbreite der vierten Inkontinenzwegwerfwindel SB4. Weiter bevorzugt gilt SB1=SB2. Weiter bevorzugt gilt SB3=SB4.

Die minimale Schrittbereichsbreite entspricht dem geringsten Abstand der seitlichen Längsränder des Schrittbereichs zueinander, gemessen in mm in der Querrichtung der Inkontinenzwegwerfwindel.

Durch eine derartige Ausbildung der jeweiligen Schrittbereichsbreite der von der Anordnung umfassten Inkontinenzwegwerfwindeln wird auf vorteilhafte Weise die Passform, der Tragekomfort und die Auslaufsicherheit der jeweiligen Inkontinenzwegwerfwindel entsprechend der Bedürfnisse von Benutzern unterschiedlicher Körpermaße und Statur verbessert. Insbesondere bei der dritten und der optionalen vierten Inkontinenzwegwerfwindel liegt der Schrittbereich besser am Körper des Benutzers an, so dass gerade bei bewegungsaktiven Benutzern die Inkontinenzwegwerfwindel zwischen den Beinen des Benutzers weniger stark aufbaut und eine Gefahr des Verrutschens reduziert ist.

In einem bevorzugten Ausführungsbeispiel weist ein Verhältnis der Beinöffnungslänge zur minimalen Schrittbereichsbreite der ersten Inkontinenzwegwerfwindel BL1/SB1 und der zweiten Inkontinenzwegwerfwindel BL2/SB2 und der dritten Inkontinenzwegwerfwindel BL3/SB3 einen Wert von 1,1-1,7, insbesondere 1,2-1,6, weiter insbesondere 1,3-1,5 auf. Weiter bevorzugt weist ein Verhältnis der Beinöffnungslänge zur minimalen Schrittbereichsbreite der vierten Inkontinenzwegwerfwindel BL4/SB4 einen Wert von 1,1-1,7, insbesondere 1,2-1,6, weiter insbesondere 1,3-1,5 auf.

Es hat sich gezeigt, dass durch ein derartiges Verhältnis der Beinöffnungslänge zur minimalen Schrittbereichsbreite auf vorteilhafte Weise die Passform jeder der von der Anordnung umfassten Inkontinenzwegwerfwindeln verbessert sein kann. Zudem können hierdurch die erste, die zweite, die dritte und die optionale vierte Inkontinenzwegwerfwindel mit vertrautem Erscheinungsbild sowie mit ähnlicher und gewohnter Handhabung und Auslaufsicherheit bereitgestellt werden.

In einem bevorzugten Ausführungsbeispiel weist die erste Inkontinenzwegwerfwindel und die zweite Inkontinenzwegwerfwindel und die dritte Inkontinenzwegwerfwindel eine jeweilige maximale Breite des Rückenbereichs und eine jeweilige maximale Breite des Vorderbereichs auf, und die maximale Breite des Rückenbereichs ist von der maximalen Breite des Vorderbereichs der jeweiligen ersten Inkontinenzwegwerfwindel und der zweiten Inkontinenzwegwerfwindel und der dritten Inkontinenzwegwerfwindel um höchstens 10%, insbesondere höchstens 7%, weiter insbesondere höchstens 5% verschieden. Weiter bevorzugt ist die maximale Breite des Rückenbereichs zu der maximalen Breite des Vorderbereichs einer jeweiligen ersten Inkontinenzwegwerfwindel und der zweiten Inkontinenzwegwerfwindel und der dritten Inkontinenzwegwerfwindel gleich.

Weiter bevorzugt weist die vierte Inkontinenzwegwerfwindel eine maximale Breite des Rückenbereichs und eine jeweilige maximale Breite des Vorderbereichs auf, und die maximale Breite des Rückenbereichs ist von der maximalen Breite des Vorderbereichs der vierten Inkontinenzwegwerfwindel um höchstens 10%, insbesondere höchstens 7%, weiter insbesondere höchstens 5% verschieden. Weiter bevorzugt ist die maximale Breite des Rückenbereichs zu der maximalen Breite des Vorderbereichs der vierten Inkontinenzwegwerfwindel gleich.

Als maximale Breite im Rückenbereich ist zu verstehen die maximale Quererstreckung der Inkontinenzwegwerfwindel gemessen in mm im Rückenbereich der Inkontinenzwegwerfwindel, also im Bereich der hinteren Seitenabschnitte oder der hinteren Windelohren. Als maximale Breite im Vorderbereich ist zu verstehen die maximale Quererstreckung der Inkontinenzwegwerfwindel gemessen in mm im Vorderbereich der Inkontinenzwegwerfwindel, also im Bereich der vorderen Seitenabschnitte oder der vorderen Windelohren.

Dadurch dass sich der Rückenbereich und der Vorderbereich jeder der von der Anordnung umfassten Inkontinenzwegwerfwindeln in ihrer maximalen Breite jeweils nicht oder nicht stark unterscheiden, weisen die von der Anordnung umfassten Inkontinenzwegwerfwindeln ein ähnliches und für den Benutzer vertrautes Erscheinungsbild und eine ähnliche Handhabung auf, was sich vorteilhaft auf das Zutrauen des Benutzers in die Inkontinenzwegwerfwindel auswirkt, das Anlegen erleichtert und den dafür benötigten Zeitaufwand verringern kann.

Zudem können die hinteren und vorderen Seitenabschnitte oder die hinteren und vorderen Windelohren einer jeweiligen Inkontinenzwegwerfwindel im Bereitstellungszustand jeweils auf gleiche Weise gefaltet sein, so dass das Auffalten für den Benutzer einfacher und weniger komplex erfolgen kann.

In einer bevorzugten Ausführungsform ist ein erstes Verhältnis der maximalen Breite des Rückenbereichs zur Beinöffnungslänge der ersten Inkontinenzwegwerfwindel RB1/BL1 größer als ein zweites Verhältnis der maximalen Breite des Rückenbereichs zur Beinöffnungslänge der zweiten Inkontinenzwegwerfwindel RB2/BL2, und das zweite Verhältnis der maximalen Breite des Rückenbereichs zur Beinöffnungslänge der zweiten Inkontinenzwegwerfwindel RB2/BL2 ist größer als ein drittes Verhältnis der maximalen Breite des Rückenbereichs zur Beinöffnungslänge der dritten Inkontinenzwegwerfwindel RB3/BL3, derart dass gilt: RB1/BL1 > RB2/BL2 und RB2/BL2 > RB3/BL3.

Weiter bevorzugt ist das dritte Verhältnis der maximalen Breite des Rückenbereichs zur Beinöffnungslänge der dritten Inkontinenzwegwerfwindel RB3/BL3 größer ist als ein viertes Verhältnis der maximalen Breite des Rückenbereichs zur Beinöffnungslänge der vierten Inkontinenzwegwerfwindel RB4/BL4, derart dass gilt: RB3/BL3 > RB4/BL4.

Mit derart ausgebildeten von der Anordnung umfassten Inkontinenzwegwerfwindeln wird dem Umstand Rechnung getragen, dass sich Benutzer mit unterschiedlicher Statur häufig stärker in einem Hüftumfang voneinander unterscheiden als in einem Umfang der Oberschenkel, so dass hierbei auf vorteilhafte Weise die Bereitstellung von Inkontinenzwegwerfwindeln mit an die physischen Bedürfnisse eines jeweiligen Benutzers angepasster Passform unterstützt wird.

In einer bevorzugten Ausführungsform umfasst die erste und die zweite und die dritte und/oder die vierte Inkontinenzwegwerfwindel eine Flüssigkeitsaufnahme- und Verteilerlage. In jeder der von der Anordnung umfassten Inkontinenzwegwerfwindeln unterstützt die Flüssigkeitsaufnahme- und Verteilerlage auf vorteilhafte Weise eine schnelle primäre Aufnahme anfallender Körperflüssigkeit und deren rasche Weiterleitung in weiter entfernte Bereiche des Absorptionskörpers.

Die Flüssigkeitsaufnahme- und Verteilerlage besteht bevorzugt aus einem Fasermaterial, insbesondere einem Vliesmaterial. Die Fasern können natürlichen oder künstlichen Ursprungs sein und können von definierter Länge (Stapelfasern) oder kontinuierlich ("endlos") sein oder in-situ gebildet werden. Die Fasern können aus einem einzelnen Polymer oder einer Polymermischung (Einkomponentenfaser) oder aus mehr als einem einzelnen Polymer und/oder einer Polymermischung (Zwei- oder Mehrkomponentenfaser) ausgebildet sein.

In einer bevorzugten Ausführungsform umfasst die Flüssigkeitsaufnahme- und Verteilerlage Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisförmigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP. Als eine Alternative ist es im Fachgebiet bekannt, ein Material aus chemisch modifizierten Zellulosefasern, beispielsweise quervernetzten Zellulosefasern, als Flüssigkeitsaufnahme- und Verteilerlage zu verwenden. Die Flüssigkeitsaufnahme- und Verteilerlage kann auch andere Materialien wie perforierte Folien oder Schäume oder dergleichen umfassen oder daraus bestehen.

Der Absorptionskörper ist geeignet und dazu bestimmt Körperausscheidungen, insbesondere Körperflüssigkeiten, insbesondere Urin aufzunehmen und dauerhaft zu speichern. Der Absorptionskörper kann hierzu vorteilhaft superabsorbierendes Polymer (SAP) enthalten, insbesondere zu 5 - 100 Gewichtsprozent, vorzugsweise zu 10 - 95 Gewichtsprozent, weiter vorzugsweise zu 15 - 90 Gewichtsprozent, weiter vorzugsweise zu 20 - 80 Gewichtsprozent, weiter vorzugsweise zu 30 - 70 Gewichtsprozent. Typischerweise kann das SAP zumindest das 15-fache, insbesondere das 20-fache seines Gewichts an 0,9 gewichtsprozentiger Kochsalzlösung absorbieren (gemessen nach NWSP 242.0.R2(15)). Das SAP kann beispielsweise partikelförmig oder faserförmig oder blatt- oder schaumförmig ausgebildet sein.

Der Absorptionskörper kann weitere Materialien, wie Cellulose (Zellstofffasern, wood pulp) oder Kunststofffasern enthalten. Denkbar ist weiterhin, den Absorptionskörper durch Anordnung von ein oder mehreren Schichten verschiedenen Materials, insbesondere aus Vliesstoff auszubilden.

In einem bevorzugten Ausführungsbeispiel umfasst der Absorptionskörper der ersten und der zweiten und der dritten und/oder der vierten Inkontinenzwegwerfwindel eine erste Saugkörperlage. In einem alternativen Ausführungsbeispiel besteht der Absorptionskörper der ersten und der zweiten und der dritten und/oder der vierten Inkontinenzwegwerfwindel aus der ersten Saugkörperlage.

Bevorzugt umfasst die erste Saugkörperlage Cellulose oder Cellulose und superabsorbierendes Polymer (SAP).

Die erste Saugkörperlage dient auf vorteilhafte Weise der dauerhaften Speicherung anfallender Körperflüssigkeiten bei jeder der von der Anordnung umfassten Inkontinenzwegwerfwindeln.

Bevorzugt umfasst der Absorptionskörper der ersten und zweiten und dritten und/oder vierten Inkontinenzwegwerfwindel eine zweite Saugkörperlage und die zweite Saugkörperlage ist zwischen der ersten Saugkörperlage und dem Backsheet angeordnet. Bevorzugt umfasst die zweite Saugkörperlage Cellulose, weiter bevorzugt besteht die zweite Saugkörperlage aus Cellulose.

Die Cellulose der zweiten Saugkörperlage verbessert die Weichheit der jeweiligen Inkontinenzwegwerfwindel von einer Backsheetseite her, insbesondere bei einer SAPhaltigen ersten Saugkörperlage, so dass auf vorteilhafte Weise bei jeder der von der Anordnung umfassten Inkontinenzwegwerfwindeln der Tragekomfort erhöht wird.

In einer bevorzugten Ausführungsform weist die erste Saugkörperlage zumindest einen in Längsrichtung erstreckten Kanal auf. Weiter bevorzugt weist die erste Saugkörperlage genau einen in Längsrichtung erstreckten Kanal auf, der bevorzugt in Querrichtung mittig, weiter bevorzugt in Querrichtung mittig angeordnet und mit geradlinigem Verlauf ausgebildet ist. Alternativ sind auch zwei oder mehr in Längsrichtung erstreckte Kanäle denkbar.

Der zumindest eine Kanal ist bevorzugt als Materialausnehmung, dabei als teilweise oder vollständig durch die erst Saugkörperlage hindurch erstreckte Materialausnehmung, ausgebildet. Alternativ kann der Kanal auch als Prägung oder Kompression ausgebildet sein. Der zumindest eine in Längsrichtung erstreckte Kanal verbessert auf vorteilhafte Weise eine schnelle Weiterleitung von im Schrittbereich anfallenden Körperflüssigkeiten hin zu weiter entfernten Bereichen des Absorptionskörpers und unterstützt daher die schnelle dauerhafte Aufnahme der Körperflüssigkeit und ein verbessertes Trockenheitsgefühl in der Gebrauchssituation bei jeder der von der Anordnung umfassten Inkontinenzwegwerfwindeln.

In einem bevorzugten Ausführungsbeispiel ist der Absorptionskörper der ersten und der zweiten und der dritten und/oder der vierten Inkontinenzwegwerfwindel sanduhrförmig ausgebildet.

Mit dem Begriff "sanduhrförmig" ist gemeint, dass der Absorptionskörper in einem hinteren Bereich und in einem vorderen Bereich in Querrichtung jeweils weiter erstreckt ist als in einem in der Längsrichtung zwischen dem hinteren und dem vorderen Bereich verorteten mittleren Bereich. Dadurch ergibt sich bei jeder der von der Anordnung umfassten Inkontinenzwegwerfwindeln eine verbesserte Auslaufsicherheit in dem hinteren Bereich und dem vorderen Bereich des Absorptionskörpers und gleichzeitig ein höherer Tragekomfort, da eine unerwünschte Anhäufung von absorbierendem Material im ohnehin schon verengten Schrittbereich vermieden werden kann.

Der Absorptionskörper ist vorzugsweise integraler Bestandteil des Hauptteils und solchen Falls herstellerseitig unlösbar mit den weiteren Komponenten des Hauptteils verbunden. Solchen Falls ist der Absorptionskörper vorzugsweise unlösbar mit einem Topsheet und/oder mit einem Backsheet des Hauptteils verbunden, welche den Absorptionskörper vorzugsweise sandwichartig umgeben.

Weiterhin kann die Inkontinenzwegwerfwindel auf der körperzugewandten Seite des Schrittbereichs beidseits jeweils eine seitliche Auslaufsperre bildende und/oder beidseits entlang einer Längserstreckung des Absorptionskörpers verlaufende Cuffelemente aufweisen. Die Cuffelemente umfassen vorzugsweise ein Vliesmaterial ("Cuffvlies"), insbesondere ein hydrophobes Vliesmaterial, oder sind daraus gebildet.

Vorzugsweise erstrecken sich Topsheet und/oder Backsheet und/oder Cuffvlies zumindest in Querrichtung, vorzugsweise auch in Längsrichtung der Inkontinenzwegwerfwindel über die Konturränder des Absorptionskörpers hinaus, um einen entsprechenden Überhang zu bilden. In dem Überhang sind Backsheet und Topsheet und/oder Cuffvlies vorzugsweise zumindest bereichsweise miteinander verbunden, insbesondere durch an sich bekannte Fügeverfahren wie Schweißen, Siegeln, Nähen oder Kleben.

In vorteilhafter Weise weist der Hauptteil in dem Schrittbereich beidseitig an den jeweiligen seitlichen freien Längsrand des Schrittbereichs angrenzend je einen in Längsrichtung der Inkontinenzwegwerfwindel erstreckten elastischen oder elastifizierten Bereich, mithin einen elastischen oder elastifizierten Beinöffnungsbereich auf. "In Längsrichtung der Inkontinenzwegwerfwindel" bedeutet hierbei, dass der elastische oder elastifizierte Beinöffnungsbereich zumindest eine in Längsrichtung der Inkontinenzwegwerfwindel verlaufende elastische Komponente aufweist, die mithin auch schräg oder gekrümmt zur Längsrichtung der Inkontinenzwegwerfwindel verlaufen kann.

Vorzugsweise sind hierzu dem Fachmann an sich bekannte elastische Fäden (beispielsweise Lycra^{®}) in vorgespanntem Zustand an den Hauptteil bildenden Materialien fixiert, vorzugsweise an Cuffvlies, oder Topsheet und/oder Backsheet des Hauptteils, insbesondere in einem Bereich, in dem Top- und/oder Backsheet und/oder Cuffvlies einen Überhang außerhalb der Konturränder des Absorptionskörpers bilden. Ein elastischer oder elastifizierter Beinöffnungsbereich kann nach einer Variante auch durch flachmaterial- oder bandförmige Materialien wie elastische Bänder, Folien, Vliesstoffe oder Schaumstoffe gebildet sein.

In einer bevorzugten Ausführungsform weisen die von der Anordnung umfassten Inkontinenzwegwerfwindeln beidseits des Absorptionskörpers jeweils einen elastifizierten Beinöffnungsbereich auf, wobei jeder der elastifizierten Beinöffnungsbereiche der ersten und zweiten Inkontinenzwegwerfwindel vorzugsweise jeweils 2 elastische Fäden umfasst und wobei jeder der elastifizierten Beinöffnungsbereiche der dritten und der optionalen vierten Inkontinenzwegwerfwindel vorzugsweise jeweils 3 elastische Fäden umfasst. Insbesondere bei der dritten und optionalen vierten Inkontinenzwegwerfwindel ergibt sich durch die Verwendung von 3 statt 2 elastischen Fäden eine erhöhte Auslaufsicherheit, da die Inkontinenzwergwerfwindel im Schrittbereich verlässlicher am Körper des Benutzers anliegen kann und die Gefahr des Verrutschens insbesondere bei erhöhter Bewegungsaktivität des Benutzers reduziert ist.

Die elastischen Fäden haben vorzugsweise eine Stärke von 300 -1500 dtex, weiter vorzugsweise 400 - 1200 dtex, weiter vorzugsweise 500 - 1000 dtex, weiter vorzugsweise 600 - 900 dtex. Aus Fertigungssicht ist es von Vorteil, bei allen von der Anordnung umfassten Inkontinenzwegwerfwindeln elastische Fäden mit gleicher Stärke, insbesondere die gleichen elastischen Fäden zu verwenden, da sich dadurch Vorteile bei der Beschaffung und Lagerhaltung und/oder der Rüstzeiten der Herstellungsmaschinen ergeben können.

Bevorzugt erstrecken sich die elastischen Fäden in allen der von der Anordnung umfassten Inkontinenzwegwerfwindeln über den gesamten jeweiligen Schrittbereich und in den jeweiligen Rückenbereich und in den jeweiligen Vorderbereich hinein. Dadurch kann sich die jeweilige Inkontinenzwegwerfwindel auf vorteilhafte Weise besser an eine Körperform des jeweiligen Benutzers im Gebrauch anpassen und die Auslaufsicherheit wie auch der Tragekomfort werden weiter verbessert.

In einer bevorzugten Ausführungsform umfasst jedes der Verschlussmittel jeweils mindestens ein Klettelement, mindestens ein Klebeelement oder eine Kombination daraus. Dabei ergibt sich auf vorteilhafte Weise eine ähnliche Handhabung der gewohnten Verschlussmittel innerhalb der Anordnung.

Bevorzugt sind die Verschlussmittel der ersten und der zweiten und der dritten und/oder der vierten Inkontinenzwegwerfwindel in Querrichtung der Inkontinenzwegwerfwindel gleich ausgebildet. Das heißt die Verschlussmittel weisen in der Querrichtung der Inkontinenzwegwerfwindel eine gleiche Anzahl, Anordnung und Erstreckung des mindestens einen Klettelements, mindestens einen Klebeelements oder der jeweiligen Klett- und/oder Klebeelemente der Kombination auf. Die Verschlussmittel der von der Anordnung umfassten Inkontinenzwegwerfwindeln können sich alternativ auch unterscheiden.

Es handelt sich bei den Verschlussmitteln typischerweise um eine Lasche aus einem ein- oder mehrschichtigen Flachmaterial, welches ausgehend von einer in der Regel um einen distalen Längsrand an einem freien Ende eines betrachteten hinteren Seitenabschnitts oder hinteren Windelohrs nach innen auf den hinteren Seitenabschnitts oder das hintere Windelohr eingefalteten Konfiguration in eine nach außen ausgefaltete Betriebsstellung entfaltbar ist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet.

Das Verschlussmittel jedes der hinteren Windelohren der jeweiligen dritten oder vierten Inkontinenzwegwerfwindel ist auf vorteilhafte Weise in der Längsrichtung des jeweiligen hinteren Windelohrs ungefähr mittig und im frei endenden distalen Abschnitt des hinteren Windelohrs vorgesehen.

In der Gebrauchssituation werden die hinteren Seitenabschnitte oder hinteren Windelohren in Überlappung mit einer körperabgewandten Seite, also der Außenseite, des Vorderbereichs des Hauptteils der jeweiligen Inkontinenzwegwerfwindel gebracht, damit die im Bereich eines jeweiligen in der Querrichtung freien Endes des hinteren Seitenabschnitts oder hinteren Windelohrs beidseits vorgesehenen Verschlussmittel auf die Außenseite des Vorderbereichs des Hauptteils geschlossen werden können. Hierzu sind die Verschlussmittel und mindestens ein Bereich der Außenseite des Vorderbereichs des Hauptteils als Verschlusssystem ausgebildet. Insbesondere weisen die Verschlussmittel hierzu mechanische Komponenten wie Kletthaken auf, insbesondere auch in Kombination mit haftklebenden Bereichen, vermittels derer die Verschlussmittel lösbar haftend mit der Außenseite des Vorderbereichs des Hauptteils in Eingriff bringbar sind. Hierzu hat es sich als vorteilhaft erwiesen, wenn die Außenseite des Vorderbereichs des Hauptteils zumindest bereichsweise, vorzugsweise vollständig durch einen entsprechend ausgebildeten Vliesstoff gebildet ist. Alternativ ist möglich, ein oder mehrere separate Klettflauschelemente auf der Außenseite des Hauptteils im Vorderbereich vorzusehen, welche als Landezone für die Verschlussmittel dienen.

Beispiel: Eine beispielhafte Anordnung umfassend eine erste, eine zweite, eine dritte und eine vierte Inkontinenzwegwerfwindel mit den in Tabelle 1 dargestellten Abmessungen. Jede der ersten, zweiten, dritten und vierten Inkontinenzwegwerfwindel in diesem Beispiel umfasst eine Längsrichtung, eine Querrichtung orthogonal zu der Längsrichtung, ein zumindest bereichsweise flüssigkeitsdurchlässiges Topsheet, ein im Wesentlichen flüssigkeitsundurchlässiges Backsheet, eine Flüssigkeitsaufnahme- und Verteilerlage, einen zwischen dem Topsheet und dem Backsheet angeordneten sanduhrförmigen Absorptionskörper mit einer ersten Saugkörperlage umfassend Cellulose und SAP und einen in Längsrichtung erstreckten Kanal, und mit einer zweiten Saugkörperlage, die zwischen der ersten Saugkörperlage und dem Backsheet angeordnet ist.

Weiter weisen die erste und die zweite Inkontinenzwegwerfwindel in diesem Beispiel einen Hauptteil umfassend einen Vorderbereich mit vorderen seitlichen Längsrändern und einen Rückenbereich mit hinteren seitlichen Längsrändern und einen in der Längsrichtung dazwischen angeordneten, zwischen den Beinen eines Benutzers zu liegen kommenden Schrittbereich auf. Die erste und die zweite Inkontinenzwegwerfwindel umfassen jeweils beidseits an den Rückenbereich angefügte und in der Querrichtung voneinander beabstandete und im Wesentlichen rechteckige hintere Seitenabschnitte, welche sich in der Querrichtung über die hinteren seitlichen Längsränder des Rückenbereichs hinauserstrecken, und jeweils beidseits an den Vorderbereich angefügte und in der Querrichtung voneinander beabstandete und im Wesentlichen rechteckige vordere Seitenabschnitte, welche sich in der Querrichtung über die vorderen seitlichen Längsränder des Vorderbereichs hinauserstrecken. Die hinteren Seitenabschnitte sind von den vorderen Seitenabschnitten in der Längsrichtung beabstandet.

Die erste und die zweite Inkontinenzwegwerfwindel weisen jeweils im Schrittbereich beidseits in Querrichtung eine Beinöffnung auf. Weiter weist in diesem Beispiel jeder der hinteren Seitenabschnitte der ersten und der zweiten Inkontinenzwegwerfwindel jeweils zwei in Längsrichtung voneinander beabstandete Verschlussmittel auf, so dass die hinteren Seitenabschnitte zum Anlegen und Schließen der jeweiligen ersten oder zweiten Inkontinenzwegwerfwindel an einen Benutzer jeweils entlang einer Umfangsrichtung um den Körper des Benutzers herumlegbar und in überlappende Anordnung mit einer Außenseite des Vorderbereiches und/oder einer Außenseite der vorderen Seitenabschnitte bringbar sind, an der sie über das jeweilige Verschlussmittel jeweils lösbar anhaftbar sind.

Die dritte und die vierte Inkontinenzwegwerfwindel in diesem Beispiel weisen jeweils einen Hauptteil umfassend einen Vorderbereich und einen Rückenbereich und einen in der Längsrichtung dazwischen angeordneten, zwischen den Beinen eines Benutzers zu liegen kommenden Schrittbereich auf. Weiter weisen die dritte und die vierte Inkontinenzwegwerfwindel jeweils im Rückenbereich beidseits in Querrichtung sich hinauserstreckende hintere Windelohren und im Vorderbereich beidseits in Querrichtung sich hinauserstreckende vordere Windelohren auf, wobei die hinteren Windelohren und die vorderen Windelohren in struktureller Einheit mit dem Hauptteil ausgebildet sind. Dabei weist jedes der beiden hinteren Windelohren jeweils ein einziges Verschlussmittel auf, so dass die hinteren Windelohren zum Anlegen und Schließen der jeweiligen dritten und vierten Inkontinenzwegwerfwindel an einen Benutzer jeweils entlang einer Umfangsrichtung um den Körper des Benutzers herumlegbar und in überlappende Anordnung mit einer Außenseite des Vorderbereiches und/oder einer Außenseite der vorderen Windelohren bringbar sind, an der sie über das jeweilige Verschlussmittel jeweils lösbar anhaftbar sind. Weiter weist die dritte und die vierte Inkontinenzwegwerfwindel in diesem Beispiel jeweils im Schrittbereich beidseits eine konturierte Beinöffnung auf.

In diesem Beispiel weisen die erste und die zweite Inkontinenzwegwerfwindel eine erste Verschlussmittellänge VL1 auf und die dritte und die vierte Inkontinenzwegwerfwindel weisen eine zweite Verschlussmittellänge VL2 auf, wobei VL2 größer ist als VL1 und wobei ein Verhältnis von VL2 zu VL1 einen Wert von 2,0 aufweist.

**Tabelle 1: Beispiel für eine Anordnung umfassend eine erste, eine zweite, eine dritte und eine vierte Inkontinenzwegwerfwindel (IWW)**

| | Maßeinheit | Erste IWW | Zweite IWW | Dritte IWW | Vierte IWW |
|---|---|---|---|---|---|
| Größenkennzeichnung | | Large (L) | Medium (M) | Small (S) | Extra small (XS) |
| Bereitgestellt für Benutzer mit Hüftumfang von | cm | >120 | 90-120 | 70-90 | <70 |
| Chassislänge | mm | 980 | 815 | 690 | 595 |
| Maximale Breite im Vorderbereich | mm | 780 | 650 | 490 | 410 |
| Maximale Breite im Rückenbereich | mm | 780 | 650 | 480 | 410 |
| Minimale Schrittbereichsbreite | mm | 320 | 320 | 230 | 230 |
| Anzahl Verschlussmittel pro Seitenabschnitt/Windelohr | | 2 | 2 | 1 | 1 |
| Erste Verschlussmittellänge VL1 (pro Verschlussmittel) | mm | 25 | 25 | | |
| Zweite Verschlussmittellänge VL2 (pro Verschlussmittel) | mm | | | 50 | 50 |
| Beinöffnungslänge der Beinöffnung | mm | 450 | 415 | | |
| Beinöffnungslänge der konturierten Beinöffnung | mm | | | 340 | 305 |
| Beinöffnungslänge / minimale Schrittbereichsbreite | | 1,41 | 1,30 | 1,48 | 1,33 |
| Chassislänge / Beinöffnungslänge | | 2,18 | 1,96 | 2,03 | 1,95 |
| Maximale Breite im Rückenbereich / Beinöffnungslänge | | 1,73 | 1,57 | 1,44 | 1,34 |

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigt:

### Zeichnungen

**Figur 1a**
   schematisch, nicht maßstäblich, eine Draufsicht auf eine erste Inkontinenzwegwerfwindel im aufgefalteten und eben ausgebreiteten Zustand
**Figur 1b**
   schematisch, nicht maßstäblich, eine Draufsicht auf eine zweite Inkontinenzwegwerfwindel im aufgefalteten und eben ausgebreiteten Zustand
**Figur 1c**
   schematisch, nicht maßstäblich, eine Draufsicht auf eine dritte Inkontinenzwegwerfwindel im aufgefalteten und eben ausgebreiteten Zustand
**Figur 1d**
   schematisch, nicht maßstäblich, eine Draufsicht auf eine vierte Inkontinenzwegwerfwindel im aufgefalteten und eben ausgebreiteten Zustand

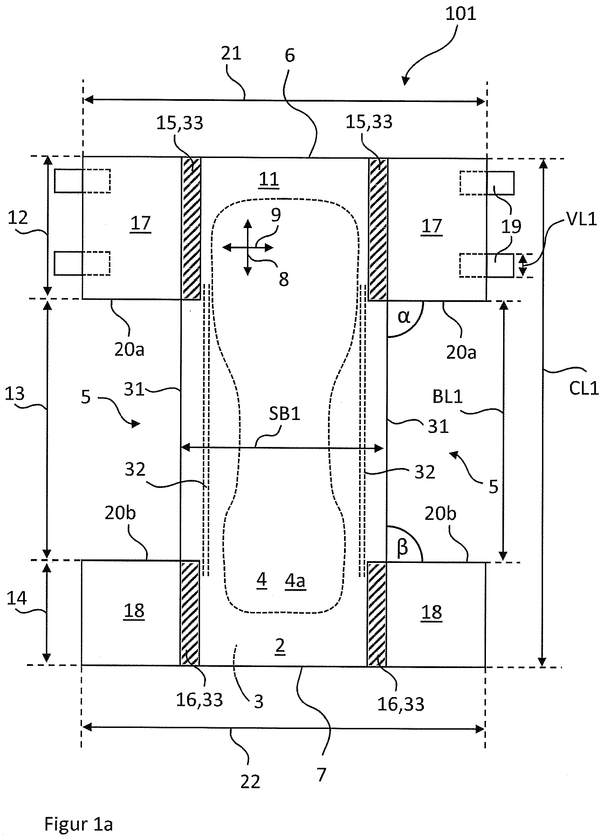

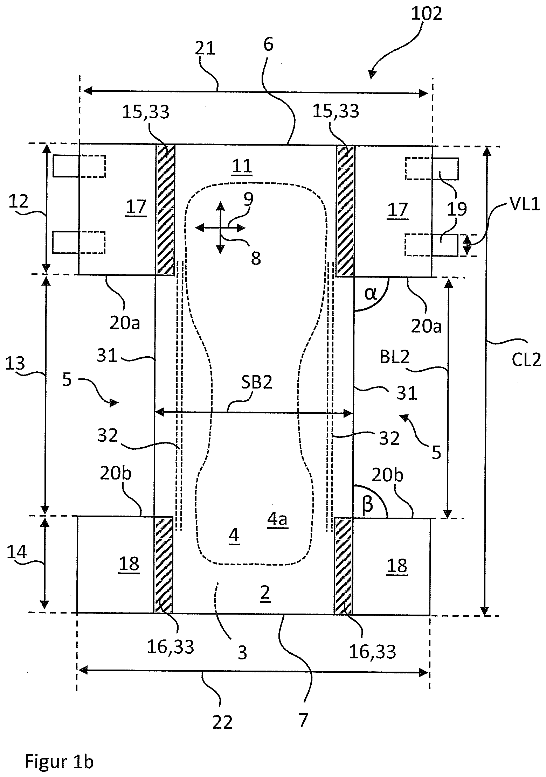

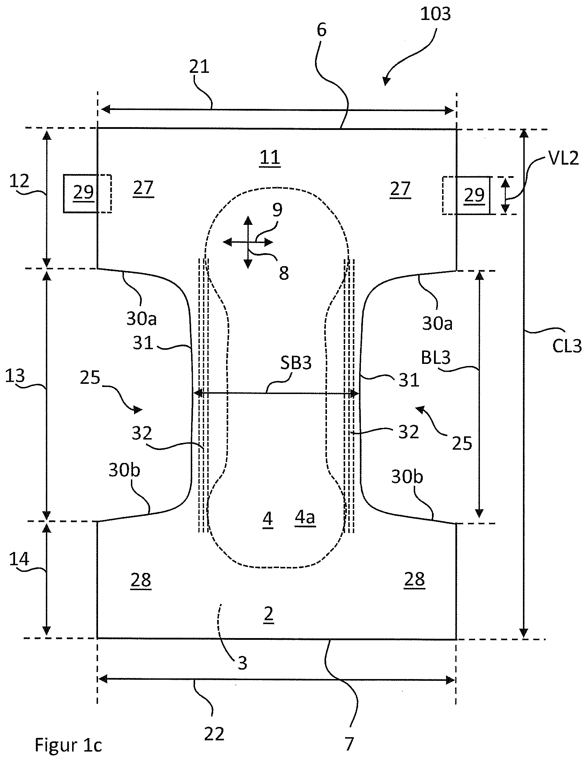

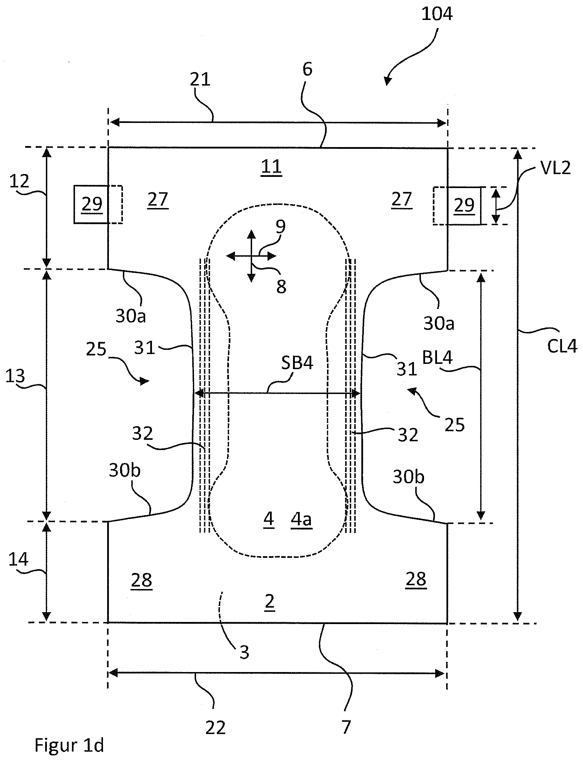

Die Figuren 1a, 1b, 1c und 1d zeigen nicht maßstäblich, sondern schematisch eine erste Inkontinenzwegwerfwindel 101, eine zweite Inkontinenzwegwerfwindel 102, eine dritte Inkontinenzwegwerfwindel 103 und eine optionale vierte Inkontinenzwegwerfwindel 104, welche gemeinsam eine beispielhafte Anordnung bilden.

Dabei zeigen die Figuren 1a und 1b eine beispielhafte erste Inkontinenzwegwerfwindel 101 und eine beispielhafte zweite Inkontinenzwegwerfwindel 102 für die Aufnahme von Körperausscheidungen. Die erste Inkontinenzwegwerfwindel 101 und die zweite Inkontinenzwegwerfwindel 102 weisen jeweils eine Längsrichtung 8 und orthogonal zu der Längsrichtung 8 eine Querrichtung 9, ein zumindest bereichsweise flüssigkeitsdurchlässiges Topsheet 2, ein im Wesentlichen flüssigkeitsundurchlässiges Backsheet 3 und einen zwischen dem Topsheet 2 und dem Backsheet 3 angeordneten sanduhrförmigen Absorptionskörper 4 auf. Die erste und die zweite Inkontinenzwegwerfwindel weisen weiter jeweils einen Hauptteil 11 mit einem Vorderbereich 14 und einem Rückenbereich 12 und einem in der Längsrichtung 8 dazwischen angeordneten, zwischen den Beinen eines Benutzers zu liegen kommenden Schrittbereich 13 auf. Beidseits sind an hintere seitliche Längsränder des Rückenbereichs 12 angefügte und in der Querrichtung 9 voneinander beabstandete und im Wesentlichen rechteckige hintere Seitenabschnitte 17 vorhanden, welche sich in der Querrichtung 9 über die hinteren seitlichen Längsränder 15 des Rückenbereichs 12 hinauserstrecken. Weiter sind jeweils beidseits an vordere seitliche Längsränder des Vorderbereichs 14 rechteckige vordere Seitenabschnitte 18 angefügt, welche in der Querrichtung 9 voneinander beabstandet sind und welche sich in der Querrichtung 9 über die vorderen seitlichen Längsränder 16 des Vorderbereichs 14 hinauserstrecken.

Die hinteren Seitenabschnitte 17 und die vorderen Seitenabschnitte 18 sind in diesem Beispiel mit dem Hauptteil 11 überlappend angefügt, wobei die hinteren Seitenabschnitte 17 innerhalb der Überlappungsbereiche 33 bevorzugt mittels Ultraschallschweißen mit dem Hauptteil 11 verbunden sind, jedoch ist jedes andere dem Fachmann an sich bekannte und zu dem Zweck geeignete Fügeverfahren, insbesondere Thermoschweißen, Nähen, Siegeln oder Kleben, ebenso möglich.

Die hinteren Seitenabschnitte 17 sind von den vorderen Seitenabschnitten 18 in der Längsrichtung 8 beabstandet. Dabei weist die erste 101 und die zweite Inkontinenzwegwerfwindel 102 im Schrittbereich 13 beidseits in Querrichtung 9 eine jeweilige Beinöffnung 5 auf.

Die Beinöffnung 5 der ersten 101 und der zweiten Inkontinenzwegwerfwindel 102 ist gebildet und begrenzt durch jeweils einen seitlichen freien Längsrand 31 des Schrittbereichs 13 und einen an diesen seitlichen freien Längsrand 31 angrenzenden inneren, also dem Schrittbereich 13 der Inkontinenzwegwerfwindel 101,102 zugewandten, Querrand 20a des jeweiligen hinteren Seitenabschnitts 17 und einen an diesen seitlichen freien Längsrand 31 angrenzenden inneren, also dem Schrittbereich 13 der Inkontinenzwegwerfwindel 101,102 zugewandten, Querrand 20b des jeweiligen vorderen Seitenabschnitts 18.

Beidseits bildet der jeweilige seitliche freie Längsrand 31 des Schrittbereichs 13 mit dem inneren Querrand 20a des hinteren Seitenabschnitts 17 einen im Wesentlichen rechten Winkel, nämlich einen Winkel α von 90°. Der jeweilige seitliche freie Längsrand 31 des Schrittbereichs 13 bildet mit dem inneren Querrand 20b des vorderen Seitenabschnitts 18 einen im Wesentlichen rechten Winkel, nämlich einen Winkel β von 90°.

Jeder der hinteren Seitenabschnitte 17 weist jeweils zwei in Längsrichtung 8 voneinander beabstandete Verschlussmittel 19 auf, so dass die hinteren Seitenabschnitte 17 zum Anlegen und Schließen der jeweiligen ersten Inkontinenzwegwerfwindel 101 oder zweiten Inkontinenzwegwerfwindel 102 an einen Benutzer jeweils entlang einer Umfangsrichtung um den Körper des Benutzers herumlegbar und in überlappende Anordnung mit einer hier nicht dargestellten Außenseite des Vorderbereiches 14 und/oder einer hier nicht dargestellten Außenseite der vorderen Seitenabschnitte 18 bringbar sind, an der sie über das jeweilige Verschlussmittel 19 jeweils lösbar anhaftbar sind. Die Verschlussmittel umfassen jeweils eine in Figuren 1a, 1b nicht dargestellte Kombination von mehreren Klett- und Klebeelementen, die in Querrichtung der Inkontinenzwegwerfwindel alternierend angeordnet sind. Zumindest eines der Verschlussmittel 19 der ersten 101 und der zweiten Inkontinenzwegwerfwindel 102 weist eine erste Verschlussmittellänge VL1 auf. In diesem Beispiel weisen alle Verschlussmittel der ersten 101 und der zweiten Inkontinenzwegwerfwindel 102 die Verschlussmittellänge VL1, mithin also die gleiche Verschlussmittellänge VL1, auf. Alternativ ist auch denkbar, dass die Verschlussmittel 19 eines jeweiligen hinteren Seitenabschnitts einer ersten 101 oder zweiten Inkontinenzwegwerfwindel 102 verschiedene Verschlussmittellängen aufweisen.

Die erste Inkontinenzwegwerfwindel 101 weist eine erste Chassislänge CL1 auf und die zweite Inkontinenzwegwerfwindel 102 weist eine zweite Chassislänge CL2 auf. Die erste Chassislänge CL1 ist in diesem Beispiel größer als die zweite Chassislänge CL2. Die Chassislänge einer Inkontinenzwegwerfwindel ist zu verstehen als maximale Längserstreckung der Inkontinenzwegwerfwindel gemessen in mm von einem hinteren Querrand 6 des Hauptteils (Chassis) 11 der Inkontinenzwegwerfwindel bis zu einem vorderen Querrand 7 des Hauptteils (Chassis) 11 der Inkontinenzwegwerfwindel.

Die Beinöffnung 5 der ersten Inkontinenzwegwerfwindel 101 weist eine erste Beinöffnungslänge BL1 auf und die Beinöffnung 5 der zweiten Inkontinenzwegwerfwindel 102 weist eine zweite Beinöffnungslänge BL2 auf, wobei BL1 größer ist als BL2.

Die erste Inkontinenzwegwerfwindel 101 weist eine minimale Schrittbereichsbreite SB1 auf und die zweite Inkontinenzwegwerfwindel 102 weist eine minimale Schrittbereichsbereite SB2 auf. In diesem Beispiel sind SB1 und SB2 gleich groß.

Die erste 101 und die zweite Inkontinenzwegwerfwindel 102 weisen eine maximale Breite des Rückenbereichs 21 auf, die, wie in der jeweiligen Figur 1a,1b ersichtlich, einer maximalen Quererstreckung der jeweiligen Inkontinenzwegwerfwindel 101,102 im Rückenbereich 12 der Inkontinenzwegwerfwindel 101,102, also im Bereich der hinteren Seitenabschnitte 17 entspricht.

Die erste 101 und die zweite Inkontinenzwegwerfwindel 102 weisen eine maximale Breite des Vorderbereichs 22 auf, die, wie in der jeweiligen Figur 1a, 1b ersichtlich, einer maximalen Quererstreckung der jeweiligen Inkontinenzwegwerfwindel 101,102 im Vorderbereich 14 der Inkontinenzwegwerfwindel 101,102, also im Bereich der vorderen Seitenabschnitte 18 entspricht. Die maximale Breite des Rückenbereichs 21 der ersten Inkontinenzwegwerfwindel 101 und der zweiten Inkontinenzwegwerfwindel 102 ist in diesem Beispiel gleich der maximalen Breite des Vorderbereichs 22 der jeweiligen ersten 101 oder zweiten Inkontinenzwegwerfwindel 102.

Ein erstes Verhältnis des maximalen Breite 21 des Rückenbereichs 12 zur Beinöffnungslänge BL1 der ersten Inkontinenzwegwerfwindel 101 RB1/BL1 ist in diesem Beispiel größer als ein zweites Verhältnis der maximalen Breite 21 des Rückenbereiches 12 zur Beinöffnungslänge BL2 der zweiten Inkontinenzwegwerfwindel 102 RB2/BL2.

Jede der ersten Inkontinenzwegwerfwindel 101 und zweiten Inkontinenzwegwerfwindel 102 weisen in diesem Beispiel im Hauptteil 11 beidseitig an den jeweiligen seitlichen freien Längsrand 31 des Schrittbereichs 13 angrenzend je einen in Längsrichtung 8 der jeweiligen Inkontinenzwegwerfwindel 101,102 erstreckten elastifizierten Beinöffnungsbereich auf, der jeweils zwei in Längsrichtung 8 der Inkontinenzwegwerfwindel 101,102 verlaufende elastische Fäden 32 mit einer Stärke von jeweils 800 dtex aufweist. Die elastischen Fäden 32 erstrecken sich über den gesamten jeweiligen Schrittbereich 13 hinweg und in den jeweiligen Rückenbereich 12 und in den jeweiligen Vorderbereich 14 hinein.

Die erste Inkontinenzwegwerfwindel 101 und die zweite Inkontinenzwegwerfwindel 102 umfasst in diesem Beispiel eine in Figuren 1a, 1b nicht dargestellte Flüssigkeitsaufnahme- und Verteilerauflage. Die Flüssigkeitsaufnahme- und Verteilerauflage ist zwischen dem Topsheet 2 und dem Absorptionskörper 4 angeordnet. Der Absorptionskörper 4 umfasst in diesem Beispiel eine erste Saugkörperlage 4a und eine nicht dargestellte zweite Saugkörperlage, wobei die zweite Saugkörperlage zwischen der ersten Saugkörperlage 4a und dem Backsheet 3 angeordnet ist. Die erste Saugkörperlage 4a umfasst in diesem Beispiel Cellulose und superabsorbierendes Polymer (SAP) und weist einen in den Figuren 1a, 1b nicht dargestellten in Längsrichtung erstreckten Kanal auf. Der Kanal ist als vollständig durch die erst Saugkörperlage hindurch erstreckte Materialausnehmung ausgebildet.

Die Figur 1c zeigt eine beispielhafte dritte Inkontinenzwegwerfwindel 103 für die Aufnahme von Körperausscheidungen. Die dritte Inkontinenzwegwerfwindel 103 weist eine Längsrichtung 8 und orthogonal zu der Längsrichtung 8 eine Querrichtung 9, ein zumindest bereichsweise flüssigkeitsdurchlässiges Topsheet 2, ein im Wesentlichen flüssigkeitsundurchlässiges Backsheet 3 und einen zwischen dem Topsheet 2 und dem Backsheet 3 angeordneten Absorptionskörper 4 auf. Die dritte Inkontinenzwegwerfwindel 103 weist weiter einen Hauptteil 11 mit einem Vorderbereich 14 und einem Rückenbereich 12 und einem in der Längsrichtung 8 dazwischen angeordneten, zwischen den Beinen eines Benutzers zu liegen kommenden Schrittbereich 13 auf. Im Rückenbereich 12 weist die dritte Inkontinenzwegwerfwindel 103 beidseits in Querrichtung 9 sich hinauserstreckende hintere Windelohren 27 und im Vorderbereich 14 beidseits in Querrichtung 9 sich hinauserstreckende vordere Windelohren 28 auf, wobei die hinteren Windelohren 27 und die vorderen Windelohren 28 in struktureller Einheit mit dem Hauptteil 11 ausgebildet sind. Jedes der beiden hinteren Windelohren 27 weist jeweils ein einziges Verschlussmittel 29 auf, so dass die hinteren Windelohren 27 zum Anlegen und Schließen der dritten Inkontinenzwegwerfwindel 103 an einen Benutzer jeweils entlang einer Umfangsrichtung um den Körper des Benutzers herumlegbar und in überlappende Anordnung mit einer hier nicht dargestellten Außenseite des Vorderbereiches 14 und/oder einer hier nicht dargestellten Außenseite der vorderen Windelohren 28 bringbar sind, an der sie über das jeweilige Verschlussmittel 29 jeweils lösbar anhaftbar sind. Im Schrittbereich 13 weist die dritte Inkontinenzwegwerfwindel 103 beidseits eine konturierte Beinöffnung 25 auf.

Dabei bilden ein innerer Querrand 30a des hinteren Windelohrs 27, ein seitlicher freien Längsrand 31 des Schrittbereichs 13 und ein innerer Querrand 30b des vorderen Windelohrs 28 gemeinsam eine kontinuierliche, stetige, gebogene Linie, gleichsam eine im Wesentlichen halbrund ausgebildete Materialaussparung im Schrittbereich 13 begrenzend. Die dritte Inkontinenzwegwerfwindel 103 erhält hierdurch eine sanduhrförmige Gestalt.

Die dritte Inkontinenzwegwerfwindel 103 weist eine dritte Chassislänge CL3 auf, die kleiner ist als die zweite Chassislänge CL2 der in Figur 1b dargestellten zweiten Inkontinenzwegwerfwindel 102. Die Chassislänge CL3 wird gemessen in mm von einem hinteren Querrand 6 des Hauptteils (Chassis) 11 der dritten Inkontinenzwegwerfwindel 103 bis zu einem vorderen Querrand 7 des Hauptteils (Chassis) 11 der dritten Inkontinenzwegwerfwindel 103.

Zumindest eines der Verschlussmittel 29 der dritten Inkontinenzwegwerfwindel 103 weist eine zweite Verschlussmittellänge VL2 auf. In diesem Beispiel weisen alle Verschlussmittel 29 der dritten Inkontinenzwegwerfwindel 103 eine gleiche Verschlussmittellänge 103 auf.

Weiter ist in diesem Beispiel die zweite Verschlussmittellänge VL2 der dritten Inkontinenzwegwerfwindel größer als die erste Verschlussmittellänge VL1 der ersten 101 und der zweiten Inkontinenzwegwerfwindel 102. VL2 ist größer als VL1 und ein Verhältnis von VL2 zu VL1 weist in diesem Beispiel einen Wert von 2,0 auf.

Die konturierte Beinöffnung 25 der dritten Inkontinenzwegwerfwindel 103 weist eine dritte Beinöffnungslänge BL3 auf, die kleiner ist als die zweite Beinöffnungslänge BL2 der Beinöffnung 5 der in Figur 1b dargestellten zweiten Inkontinenzwegwerfwindel 102.

Die dritte Inkontinenzwegwerfwindel 103 weist eine minimale Schrittbereichsbreite SB3 auf, die in diesem Beispiel kleiner ist als die jeweilige minimale Schrittbreite SB1,SB2 der in Figuren 1a und 1b dargestellten ersten 101 und zweiten Inkontinenzwegwerfwindel 102. Die dritte Inkontinenzwegwerfwindel 103 weist eine maximale Breite des Rückenbereichs 22 auf, die, wie in der jeweiligen Figur 1c ersichtlich, einer maximalen Quererstreckung der dritten Inkontinenzwegwerfwindel 103 im Rückenbereich 12 der dritten Inkontinenzwegwerfwindel 103 also im Bereich der hinteren Windelohren 27 entspricht.

Die dritte Inkontinenzwegwerfwindel 103 weist eine maximale Breite des Vorderbereichs 22 auf, die, wie in der jeweiligen Figur 1c ersichtlich, einer maximalen Quererstreckung der dritten Inkontinenzwegwerfwindel 103 im Vorderbereich 14 der dritten Inkontinenzwegwerfwindel 103 also im Bereich der vorderen Windelohren 28 entspricht. Die maximale Breite des Rückenbereichs 21 der dritten Inkontinenzwegwerfwindel 103 ist in diesem Beispiel gleich der maximalen Breite des Vorderbereichs 22 der dritten Inkontinenzwegwerfwindel 103.

Das zweite Verhältnis der maximalen Breite 21 des Rückenbereichs 12 zur Beinöffnungslänge BL2 der in Figur 1b dargestellten zweiten Inkontinenzwegwerfwindel 102 RB2/BL2 ist in diesem Beispiel größer als ein drittes Verhältnis der maximalen Breite 21 des Rückenbereiches 12 zur Beinöffnungslänge BL3 der dritten Inkontinenzwegwerfwindel 103 RB3/BL3.

Die dritte Inkontinenzwegwerfwindel 103 weist in diesem Beispiel im Hauptteil 11 beidseitig an den jeweiligen seitlichen freien Längsrand 31 des Schrittbereichs 13 angrenzend je einen in Längsrichtung 8 der dritten Inkontinenzwegwerfwindel 103 erstreckten elastifizierten Beinöffnungsbereich auf, der jeweils drei in Längsrichtung 8 der dritten Inkontinenzwegwerfwindel 103 verlaufende elastische Fäden 32 mit einer Stärke von jeweils 800 dtex aufweist. Die elastischen Fäden 32 erstrecken sich über den gesamten jeweiligen Schrittbereich 13 hinweg und in den jeweiligen Rückenbereich 12 und in den jeweiligen Vorderbereich 14 hinein.

Die dritte Inkontinenzwegwerfwindel 103 umfasst in diesem Beispiel eine in Figur 1c nicht dargestellte Flüssigkeitsaufnahme- und Verteilerauflage. Die Flüssigkeitsaufnahme- und Verteilerauflage ist zwischen dem Topsheet 2 und dem Absorptionskörper 4 angeordnet. Der Absorptionskörper 4 umfasst in diesem Beispiel eine erste Saugkörperlage 4a und eine nicht dargestellte zweite Saugkörperlage, wobei die zweite Saugkörperlage zwischen der ersten Saugkörperlage 4a und dem Backsheet 3 angeordnet ist. Die erste Saugkörperlage 4a umfasst in diesem Beispiel Cellulose und superabsorbierendes Polymer (SAP) und weist einen in Figur 1c nicht dargestellten in Längsrichtung erstreckten Kanal auf. Der Kanal ist als vollständig durch die erst Saugkörperlage hindurch erstreckte Materialausnehmung ausgebildet.

Die in Figur 1d gezeigte beispielhafte vierte Inkontinenzwegwerfwindel 104 weist die Merkmale der in Figur 1c dargestellten und vorstehend beschriebenen dritten Inkontinenzwegwerfwindel 103 mit den folgenden Abweichungen auf. Abweichend von der dritten Inkontinenzwegwerfwindel 103 weist die vierte Inkontinenzwegwerfwindel 104 eine vierte Chassislänge CL4 auf, die kleiner ist als die dritte Chassislänge CL3 der dritten Inkontinenzwegwerfwindel 103. Die Chassislänge CL4 wird gemessen wir oben mit Bezug zu Figuren 1a,1b und1c beschrieben.

Darüber hinaus weist die vierte Inkontinenzwegwerfwindel 104 eine Beinöffnungslänge BL4 der konturierten Beinöffnung 25 auf, und die Beinöffnungslänge BL4 ist kleiner als die Beinöffnungslänge BL3 der konturierten Beinöffnung der dritten Inkontinenzwegwerfwindel 103.

Eine maximale Breite des Rückenbereichs 21 der vierten Inkontinenzwegwerfwindel 104 ist in diesem Beispiel gleich einer maximalen Breite des Vorderbereichs 22 der vierten Inkontinenzwegwerfwindel 104.

Das oben beschriebene dritte Verhältnis der maximalen Breite 21 des Rückenbereichs 12 zur Beinöffnungslänge BL3 der in Figur 1c dargestellten dritten Inkontinenzwegwerfwindel 103 RB3/BL3 ist in diesem Beispiel größer als ein viertes Verhältnis der maximalen Breite 21 des Rückenbereiches 12 zur Beinöffnungslänge BL4 der vierten Inkontinenzwegwerfwindel 104 RB4/BL4.

Die vierte Inkontinenzwegwerfwindel 104 weist eine minimale Schrittbereichsbreite SB4 auf, die in diesem Beispiel kleiner ist als die jeweilige minimale Schrittbreite SB1,SB2 der in Figuren 1a und 1b dargestellten ersten 101 und zweiten Inkontinenzwegwerfwindel 102, und gleich groß wie die minimalen Schrittbereichsbreite der in Figur 1c dargestellten dritten Inkontinenzwegwerfwindel 103.

Die vierte Inkontinenzwegwerfwindel 104 umfasst in diesem Beispiel eine in Figur 1d nicht dargestellte Flüssigkeitsaufnahme- und Verteilerauflage. Die Flüssigkeitsaufnahme- und Verteilerauflage ist zwischen dem Topsheet 2 und dem Absorptionskörper 4 angeordnet. Der Absorptionskörper 4 umfasst in diesem Beispiel eine erste Saugkörperlage 4a und eine nicht dargestellte zweite Saugkörperlage, wobei die zweite Saugkörperlage zwischen der ersten Saugkörperlage 4a und dem Backsheet 3 angeordnet ist. Die erste Saugkörperlage 4a umfasst in diesem Beispiel Cellulose und superabsorbierendes Polymer (SAP) und weist einen in der Figur 1d nicht dargestellten in Längsrichtung erstreckten Kanal auf. Der Kanal ist als vollständig durch die erst Saugkörperlage hindurch erstreckte Materialausnehmung ausgebildet.

## Patentansprüche

1. Anordnung umfassend zumindest eine erste Inkontinenzwegwerfwindel (101) und eine zweite Inkontinenzwegwerfwindel (102) und eine dritte Inkontinenzwegwerfwindel (103), wobei die Anordnung jeweils zumindest einen Vertreter der jeweiligen ersten, zweiten und dritten Inkontinenzwegwerfwindel enthaltenden Packungen aufweist, wobei die erste Inkontinenzwegwerfwindel (101) eine erste Chassislänge CL1, die zweite Inkontinenzwegwerfwindel (102) eine zweite Chassislänge CL2 und die dritte Inkontinenzwegwerfwindel (103) eine dritte Chassislänge CL3 aufweist,
- wobei die erste (101), die zweite (102) und die dritte Inkontinenzwegwerfwindel (103) jeweils eine Längsrichtung (8) und orthogonal zu der Längsrichtung (8) eine Querrichtung (9) aufweisen und
- wobei die erste (101), die zweite (102) und die dritte Inkontinenzwegwerfwindel (103) jeweils ein zumindest bereichsweise flüssigkeitsdurchlässiges Topsheet (2), ein im Wesentlichen flüssigkeitsundurchlässiges Backsheet (3) und einen zwischen dem Topsheet (2) und dem Backsheet (3) angeordneten Absorptionskörper (4) umfassen,
- wobei gilt CL1>CL2 und CL2>CL3,
- wobei die erste Inkontinenzwegwerfwindel (101) und die zweite Inkontinenzwegwerfwindel (102) und die dritte Inkontinenzwegwerfwindel (103) jeweils einen Hauptteil (11) umfassend einen Vorderbereich (14) und einen Rückenbereich (12) und einen in der Längsrichtung (8) dazwischen angeordneten, zwischen den Beinen eines Benutzers zu liegen kommenden Schrittbereich (13) umfasst,
- und wobei die erste (101) und die zweite Inkontinenzwegwerfwindel (102) jeweils beidseits an hintere seitliche Längsränder (15) des Rückenbereichs (12) angefügte und in der Querrichtung (9) voneinander beabstandete und im Wesentlichen rechteckige hintere Seitenabschnitte (17) umfassen, welche sich in der Querrichtung (9) über die hinteren seitlichen Längsränder (15) des Rückenbereichs (12) hinauserstrecken,
- und wobei die erste (101) und die zweite Inkontinenzwegwerfwindel (102) jeweils beidseits an vordere seitliche Längsränder (16) des Vorderbereichs (14) angefügte und in der Querrichtung (9) voneinander beabstandete und im Wesentlichen rechteckige vordere Seitenabschnitte (18) umfassen, welche sich in der Querrichtung (9) über die vorderen seitlichen Längsränder (16) des Vorderbereichs (14) hinauserstrecken,
- und wobei die hinteren Seitenabschnitte (17) von den vorderen Seitenabschnitten (18) in der Längsrichtung (8) beabstandet sind,
- wobei die erste (101) und die zweite Inkontinenzwegwerfwindel (102) im Schrittbereich (13) beidseits in Querrichtung (9) eine Beinöffnung (5) aufweist,
- wobei jeder der hinteren Seitenabschnitte (17) jeweils zwei in Längsrichtung (8) voneinander beabstandete Verschlussmittel (19) aufweist, so dass die hinteren Seitenabschnitte (17) zum Anlegen und Schließen der jeweiligen ersten (101) oder zweiten Inkontinenzwegwerfwindel (102) an einen Benutzer jeweils entlang einer Umfangsrichtung um den Körper des Benutzers herumlegbar und in überlappende Anordnung mit einer Außenseite des Vorderbereiches (14) und/oder einer Außenseite der vorderen Seitenabschnitte (18) bringbar sind, an der sie über das jeweilige Verschlussmittel (19) jeweils lösbar anhaftbar sind,
- wobei die dritte Inkontinenzwegwerfwindel (103) im Rückenbereich (12) beidseits in Querrichtung (9) sich hinauserstreckende hintere Windelohren (27) und im Vorderbereich (14) beidseits in Querrichtung (9) sich hinauserstreckende vordere Windelohren (28) aufweist, wobei die hinteren Windelohren (27) und die vorderen Windelohren (28) in struktureller Einheit mit dem Hauptteil (11) ausgebildet sind,
- wobei jedes der beiden hinteren Windelohren (27) jeweils ein einziges Verschlussmittel (29) aufweist, so dass die hinteren Windelohren (27) zum Anlegen und Schließen der dritten Inkontinenzwegwerfwindel (103) an einen Benutzer jeweils entlang einer Umfangsrichtung um den Körper des Benutzers herumlegbar und in überlappende Anordnung mit einer Außenseite des Vorderbereiches (14) und/oder einer Außenseite der vorderen Windelohren (28) bringbar sind, an der sie über das jeweilige Verschlussmittel (29) jeweils lösbar anhaftbar sind,
- und wobei die dritte Inkontinenzwegwerfwindel (103) im Schrittbereich (13) beidseits eine konturierte Beinöffnung (25) aufweist.

2. Anordnung nach Anspruch 1 umfassend eine vierte Inkontinenzwegwerfwindel (104) mit einer Chassislänge CL4, einer Längsrichtung (8) , einer Querrichtung (9) orthogonal zu der Längsrichtung (8), einem zumindest bereichsweise flüssigkeitsdurchlässigen Topsheet (2), einem im Wesentlichen flüssigkeitsundurchlässigen Backsheet (3) und einem zwischen dem Topsheet (2) und dem Backsheet (3) angeordneten Absorptionskörper (4),
- wobei gilt CL3>CL4,
- wobei die vierte Inkontinenzwegwerfwindel (104) einen Hauptteil (11) umfassend einen Vorderbereich (14) und einen Rückenbereich (12) und einen in der Längsrichtung (8) dazwischen angeordneten, zwischen den Beinen eines Benutzers zu liegen kommenden Schrittbereich (13) umfasst,
- wobei die vierte Inkontinenzwegwerfwindel (104) im Rückenbereich (12) beidseits in Querrichtung (9) sich hinauserstreckende hintere Windelohren (27) und im Vorderbereich (14) beidseits in Querrichtung (9) sich hinauserstreckende vordere Windelohren (28) aufweist, wobei die hinteren Windelohren (27) und die vorderen Windelohren (28) in struktureller Einheit mit dem Hauptteil (11) ausgebildet sind,
- wobei jedes der beiden hinteren Windelohren (27) jeweils ein einziges Verschlussmittel (29) aufweist, so dass die hinteren Windelohren (27) zum Anlegen und Schließen der vierten Inkontinenzwegwerfwindel (104) an einen Benutzer jeweils entlang einer Umfangsrichtung um den Körper des Benutzers herumlegbar und in überlappende Anordnung mit einer Außenseite des Vorderbereiches (14) und/oder einer Außenseite der vorderen Windelohren (28) bringbar sind, an der sie über das jeweilige Verschlussmittel (29) jeweils lösbar anhaftbar sind,
- und wobei die vierte Inkontinenzwegwerfwindel (104) im Schrittbereich (13) beidseits eine konturierte Beinöffnung (25) aufweist.

3. Anordnung nach einem der vorhergehenden Ansprüche, wobei mindestens eines der Verschlussmittel (19) der ersten (101) und/oder der zweiten Inkontinenzwegwerfwindel (102) eine erste Verschlussmittellänge VL1 in der Längsrichtung (8) der Inkontinenzwegwerfwindel aufweist und wobei mindestens eines der Verschlussmittel (19) der dritten (103) und/oder der vierten Inkontinenzwegwerfwindel (104) eine zweite Verschlussmittellänge VL2 in der Längsrichtung (8) der Inkontinenzwegwerfwindel aufweist, und wobei gilt VL1<VL2.

4. Anordnung nach Anspruch 3, wobei ein Verhältnis von VL2 zu VL1 einen Wert von 1,2-3,0, insbesondere 1,3-2,7, weiter insbesondere 1,5-2,5, weiter insbesondere 1,8-2,2, weiter insbesondere von 2,0 aufweist.

5. Anordnung nach einem der vorhergehenden Ansprüche, wobei die Beinöffnung (5) der ersten Inkontinenzwegwerfwindel (101) eine erste Beinöffnungslänge BL1 aufweist, und wobei die Beinöffnung (5) der zweiten Inkontinenzwegwerfwindel (102) eine zweite Beinöffnungslänge BL2 aufweist, und wobei die konturierte Beinöffnung (25) der dritten Inkontinenzwegwerfwindel (103) eine dritte Beinöffnungslänge BL3 aufweist, und wobei insbesondere gilt BL1>BL2 und BL2>BL3.

6. Anordnung nach Anspruch 5, wobei die konturierte Beinöffnung (25) der vierten Inkontinenzwegwerfwindel (104) eine vierte Beinöffnungslänge BL4 aufweist, und wobei insbesondere gilt BL3>BL4.

7. Anordnung nach einem der Ansprüche 5 oder 6, wobei ein Verhältnis der Chassislänge zur Beinöffnungslänge der ersten Inkontinenzwegwerfwindel (101) CL1/BL1 und der zweiten Inkontinenzwegwerfwindel (102) CL2/BL2 und der dritten Inkontinenzwegwerfwindel (103) CL3/BL3 jeweils einen Wert von 1,6-2,4, insbesondere von 1,7-2,3, weiter insbesondere von 1,8-2,2, weiter insbesondere 1,9-2,2 aufweist.

8. Anordnung nach einem der Ansprüche 6 oder 7, wobei ein Verhältnis der Chassislänge zur Beinöffnungslänge der vierten Inkontinenzwegwerfwindel (104) CL4/BL4 einen Wert von 1,6-2,4, insbesondere von 1,7-2,3, weiter insbesondere von 1,8-2,2, weiter insbesondere 1,9-2,2 aufweist.

9. Anordnung nach einem der Ansprüche 5 bis 8, wobei die Beinöffnungslänge der Beinöffnung (5) der ersten Inkontinenzwegwerfwindel (101) BL1 und der zweiten Inkontinenzwegwerfwindel (102) BL2 einen Wert von 380-490 mm, insbesondere 390-480 mm, weiter insbesondere 400-470 mm, weiter insbesondere 410-460 mm aufweist.

10. Anordnung nach einem der Ansprüche 5 bis 9, wobei die Beinöffnungslänge der konturierten Beinöffnung (25) der dritten Inkontinenzwegwerfwindel (103) BL3 einen Wert von 280-370 mm, insbesondere von 290-360 mm, weiter insbesondere von 300-350 mm aufweist.

11. Anordnung nach einem der Ansprüche 6 bis 10, wobei die Beinöffnungslänge der konturierten Beinöffnung (25) der vierten Inkontinenzwegwerfwindel (104) BL4 einen Wert von 280-370 mm, insbesondere von 290-360 mm, weiter insbesondere von 300-350 mm aufweist.

12. Anordnung nach einem der vorhergehenden Ansprüche, wobei eine minimale Schrittbereichsbreite der ersten Inkontinenzwegwerfwindel (101) SB1 und eine minimale Schrittbereichsbreite der zweiten Inkontinenzwegwerfwindel (102) SB2 jeweils größer ist als eine minimale Schrittbereichsbreite der dritten Inkontinenzwegwerfwindel (103) SB3.

13. Anordnung nach Anspruch 12, wobei die minimale Schrittbereichsbreite der ersten Inkontinenzwegwerfwindel (101) SB1 und die minimale Schrittbereichsbreite der zweiten Inkontinenzwegwerfwindel (102) SB2 jeweils größer ist als eine minimale Schrittbereichsbreite der vierten Inkontinenzwegwerfwindel (104) SB4, wobei insbesondere gilt SB1=SB2, weiter insbesondere gilt SB3=SB4.

14. Anordnung nach einem der Ansprüche 12 oder 13, wobei ein Verhältnis der Beinöffnungslänge zur minimalen Schrittbereichsbreite der ersten Inkontinenzwegwerfwindel (101) BL1/SB1 und der zweiten Inkontinenzwegwerfwindel (102) BL2/SB2 und der dritten Inkontinenzwegwerfwindel (103) BL3/SB3 einen Wert von 1,1-1,7, insbesondere 1,2-1,6, weiter insbesondere 1,3-1,5 aufweist.

15. Anordnung nach einem der Ansprüche 13 oder 14, wobei ein Verhältnis der Beinöffnungslänge zur minimalen Schrittbereichsbreite der vierten Inkontinenzwegwerfwindel (104) BL4/SB4 einen Wert von 1,1-1,7, insbesondere 1,2-1,6, weiter insbesondere 1,3-1,5 aufweist.

16. Anordnung nach einem der vorhergehenden Ansprüche, wobei die erste Inkontinenzwegwerfwindel (101) und die zweite Inkontinenzwegwerfwindel (102) und die dritte Inkontinenzwegwerfwindel (103) eine jeweilige maximale Breite des Rückenbereichs (21) und eine jeweilige maximale Breite des Vorderbereichs (22) aufweisen, und wobei die maximale Breite des Rückenbereichs (21) von der maximalen Breite des Vorderbereichs (22) der jeweiligen ersten Inkontinenzwegwerfwindel (101) und der zweiten Inkontinenzwegwerfwindel (102) und der dritten Inkontinenzwegwerfwindel (103) um höchstens 10%, insbesondere höchstens 7%, weiter insbesondere höchstens 5% verschieden ist, wobei weiter insbesondere die maximale Breite des Rückenbereichs (21) zu der maximalen Breite des Vorderbereichs (22) einer jeweiligen ersten Inkontinenzwegwerfwindel (101) und der zweiten Inkontinenzwegwerfwindel (102) und der dritten Inkontinenzwegwerfwindel (103) gleich ist.

17. Anordnung nach einem der vorhergehenden Ansprüche 2 bis 16, wobei die vierte Inkontinenzwegwerfwindel (104) eine maximale Breite des Rückenbereichs (21) und eine jeweilige maximale Breite des Vorderbereichs (22) aufweist, und wobei die maximale Breite des Rückenbereichs (21) von der maximalen Breite des Vorderbereichs (22) der vierten Inkontinenzwegwerfwindel (104) um höchstens 10%, insbesondere höchstens 7%, weiter insbesondere höchstens 5% verschieden ist, wobei weiter insbesondere die maximale Breite des Rückenbereichs (21) zu der maximalen Breite des Vorderbereichs (22) der vierten Inkontinenzwegwerfwindel (104) gleich ist.

18. Anordnung nach einem der Ansprüche 16 oder 17, wobei ein erstes Verhältnis der maximalen Breite des Rückenbereichs (21) zur Beinöffnungslänge der ersten Inkontinenzwegwerfwindel (101) RB1/BL1 größer ist als ein zweites Verhältnis der maximalen Breite des Rückenbereichs (21) zur Beinöffnungslänge der zweiten Inkontinenzwegwerfwindel (102) RB2/BL2, und wobei das zweite Verhältnis der maximalen Breite des Rückenbereichs (21) zur Beinöffnungslänge der zweiten Inkontinenzwegwerfwindel (102) RB2/BL2 größer ist als ein drittes Verhältnis der maximalen Breite des Rückenbereichs (21) zur Beinöffnungslänge der dritten Inkontinenzwegwerfwindel (103) RB3/BL3, derart dass gilt: RB1/BL1 > RB2/BL2 und RB2/BL2 > RB3/BL3.

19. Anordnung nach einem der vorhergehenden Ansprüche, wobei die erste und die zweite und die dritte und/oder die vierte Inkontinenzwegwerfwindel eine Flüssigkeitsaufnahme- und Verteilerlage umfasst.

20. Anordnung nach einem der vorhergehenden Ansprüche, wobei der Absorptionskörper (4) der ersten (101) und der zweiten (102) und der dritten (103) und/oder der vierten Inkontinenzwegwerfwindel (104) eine erste Saugkörperlage (4a) umfasst oder daraus besteht und wobei die erste Saugkörperlage (4a) bevorzugt Cellulose oder Cellulose und superabsorbierendes Polymer (SAP) umfasst.

21. Anordnung nach Anspruch 20, wobei der Absorptionskörper der ersten und zweiten und dritten und/oder vierten Inkontinenzwegwerfwindel eine zweite Saugkörperlage umfasst und wobei die zweite Saugkörperlage zwischen der ersten Saugkörperlage und dem Backsheet angeordnet ist.

22. Anordnung nach einem der Ansprüche 20 oder 21, wobei die erste Saugkörperlage zumindest einen in Längsrichtung erstreckten Kanal aufweist.

## Claims

1. Arrangement comprising at least a first disposable incontinence diaper (101) and a second disposable incontinence diaper (102) and a third disposable incontinence diaper (103), wherein the arrangement has in each case at least one representative of the respective first, second and third disposable incontinence diapers containing packs, wherein the first disposable incontinence diaper (101) has a first chassis length CL1, the second disposable incontinence diaper (102) has a second chassis length CL2 and the third disposable incontinence diaper (103) has a third chassis length CL3,
- wherein the first (101), the second (102) and the third disposable incontinence diaper (103) each have a longitudinal direction (8) and a transverse direction (9) orthogonal to the longitudinal direction (8), and
- wherein the first (101), the second (102) and the third disposable incontinence diaper (103) each comprise an at least partially liquid-permeable topsheet (2), a substantially liquid-impermeable backsheet (3) and an absorption body (4) arranged between the topsheet (2) and the backsheet (3),
- wherein CL1>CL2 and CL2>CL3,
- wherein the first disposable incontinence diaper (101) and the second disposable incontinence diaper (102) and the third disposable incontinence diaper (103) each comprise a main part (11) comprising a front region (14) and a back region (12) and a crotch region (13) arranged in between in the longitudinal direction (8) and coming to lie between a user's legs,
- and wherein the first (101) and the second disposable incontinence diaper (102) each comprise substantially rectangular rear side portions (17) which are attached on both sides to rear lateral longitudinal edges (15) of the back region (12) and are spaced apart from one another in the transverse direction (9) and extend in the transverse direction (9) beyond the rear lateral longitudinal edges (15) of the back region (12),
- and wherein the first (101) and the second disposable incontinence diaper (102) each comprise substantially rectangular front side portions (18) which are attached on both sides to front lateral longitudinal edges (16) of the front region (14) and are spaced apart from one another in the transverse direction (9) and extend in the transverse direction (9) beyond the front lateral longitudinal edges (16) of the front region (14),
- and wherein the rear side portions (17) are spaced apart from the front side portions (18) in the longitudinal direction (8),
- wherein the first (101) and the second disposable incontinence diaper (102) have a leg opening (5) in the crotch region (13) on both sides in the transverse direction (9),
- wherein each of the rear side portions (17) has in each case two closure means (19), which are spaced apart from one another in the longitudinal direction (8), such that the rear side portions (17) for placing the respective first (101) or second disposable incontinence diaper (102) onto a user and closing same can be laid around the user's body in each case along a circumferential direction and can be brought into an overlapping configuration with an outer side of the front region (14) and/or an outer side of the front side portions (18), to which they can each be detachably attached via the respective closure means (19),
- wherein the third disposable incontinence diaper (103) has, in the back region (12), rear diaper ears (27) extending therebeyond on both sides in the transverse direction (9) and, in the front region (14), front diaper ears (28) extending therebeyond on both sides in the transverse direction (9), wherein the rear diaper ears (27) and the front diaper ears (28) are formed as a structural unit with the main part (11),
- wherein each of the two rear diaper ears (27) in each case has a single closure means (29), such that the rear diaper ears (27) for placing the third disposable incontinence diaper (103) onto a user and closing same can be laid around the user's body in each case along a circumferential direction and can be brought into an overlapping configuration with an outer side of the front region (14) and/or an outer side of the front diaper ears (28), to which they can each be detachably attached via the respective closure means (29),
- and wherein the third disposable incontinence diaper (103) has a contoured leg opening (25) on both sides in the crotch region (13).

2. Arrangement according to Claim 1, comprising a fourth disposable incontinence diaper (104) having a chassis length CL4, a longitudinal direction (8), a transverse direction (9) orthogonal to the longitudinal direction (8), an at least partially liquid-permeable topsheet (2), a substantially liquid-impermeable backsheet (3) and an absorption body (4) arranged between the topsheet (2) and the backsheet (3),
- wherein CL3>CL4,
- wherein the fourth disposable incontinence diaper (104) comprises a main part (11) comprising a front region (14) and a back region (12) and a crotch region (13) arranged in between in the longitudinal direction (8) and coming to lie between a user's legs,
- wherein the fourth disposable incontinence diaper (104) has, in the back region (12), rear diaper ears (27) extending therebeyond on both sides in the transverse direction (9) and, in the front region (14), front diaper ears (28) extending therebeyond on both sides in the transverse direction (9), wherein the rear diaper ears (27) and the front diaper ears (28) are formed as a structural unit with the main part (11),
- wherein each of the two rear diaper ears (27) in each case has a single closure means (29), such that the rear diaper ears (27) for placing the fourth disposable incontinence diaper (104) onto a user and closing same can be laid around the user's body in each case along a circumferential direction and can be brought into an overlapping configuration with an outer side of the front region (14) and/or an outer side of the front diaper ears (28), to which they can each be detachably attached via the respective closure means (29),
- and wherein the fourth disposable incontinence diaper (104) has a contoured leg opening (25) on both sides in the crotch region (13).

3. Arrangement according to either of the preceding claims, wherein at least one of the closure means (19) of the first (101) and/or the second disposable incontinence diaper (102) has a first closure means length VL1 in the longitudinal direction (8) of the disposable incontinence diaper, and wherein at least one of the closure means (19) of the third (103) and/or the fourth disposable incontinence diaper (104) has a second closure means length VL2 in the longitudinal direction (8) of the disposable incontinence diaper, and wherein VL1<VL2.

4. Arrangement according to Claim 3, wherein a ratio of VL2 to VL1 has a value of 1.2-3.0, in particular 1.3-2.7, more particularly 1.5-2.5, more particularly 1.8-2.2, more particularly 2.0.

5. Arrangement according to one of the preceding claims, wherein the leg opening (5) of the first disposable incontinence diaper (101) has a first leg opening length BL1, and wherein the leg opening (5) of the second disposable incontinence diaper (102) has a second leg opening length BL2, and wherein the contoured leg opening (25) of the third disposable incontinence diaper (103) has a third leg opening length BL3, and wherein, in particular, BL1>BL2 and BL2>BL3.

6. Arrangement according to Claim 5, wherein the contoured leg opening (25) of the fourth disposable incontinence diaper (104) has a fourth leg opening length BL4, and wherein, in particular, BL3>BL4.

7. Arrangement according to either of Claims 5 and 6, wherein a ratio of the chassis length to the leg opening length of the first disposable incontinence diaper (101) CL1/BL1 and the second disposable incontinence diaper (102) CL2/BL2 and the third disposable incontinence diaper (103) CL3/BL3 in each case has a value of 1.6-2.4, more particularly of 1.7-2.3, more particularly of 1.8-2.2, more particularly 1.9-2.2.

8. Arrangement according to either of Claims 6 and 7, wherein a ratio of the chassis length to the leg opening length of the fourth disposable incontinence diaper (104) CL4/BL4 has a value of 1.6-2.4, in particular of 1.7-2.3, more particularly of 1.8-2.2, more particularly of 1.9-2.2.

9. Arrangement according to one of Claims 5 to 8, wherein the leg opening length of the leg opening (5) of the first disposable incontinence diaper (101) BL1 and of the second disposable incontinence diaper (102) BL2 has a value of 380-490 mm, in particular 390-480 mm, more particularly 400-470 mm, more particularly 410-460 mm.

10. Arrangement according to one of Claims 5 to 9, wherein the leg opening length of the contoured leg opening (25) of the third disposable incontinence diaper (103) BL3 has a value of 280-370 mm, in particular of 290-360 mm, more particularly of 300-350 mm.

11. Arrangement according to one of Claims 6 to 10, wherein the leg opening length of the contoured leg opening (25) of the fourth disposable incontinence diaper (104) BL4 has a value of 280-370 mm, in particular of 290-360 mm, more particularly of 300-350 mm.

12. Arrangement according to one of the preceding claims, wherein a minimum crotch region width of the first disposable incontinence diaper (101) SB1 and a minimum crotch region width of the second disposable incontinence diaper (102) SB2 is in each case larger than a minimum crotch region width of the third disposable incontinence diaper (103) SB3.

13. Arrangement according to Claim 12, wherein the minimum crotch region width of the first disposable incontinence diaper (101) SB1 and the minimum crotch region width of the second disposable incontinence diaper (102) SB2 is in each case larger than a minimum crotch region width of the fourth disposable incontinence diaper (104) SB4, wherein in particular SB1=SB2, more particularly SB3=SB4.

14. Arrangement according to either of Claims 12 and 13, wherein a ratio of the leg opening length to the minimum crotch region width of the first disposable incontinence diaper (101) BL1/SB1 and the second disposable incontinence diaper (102) BL2/SB2 and the third disposable incontinence diaper (103) BL3/SB3 has a value of 1.1-1.7, in particular 1.2-1.6, more particularly 1.3-1.5.

15. Arrangement according to either of Claims 13 and 14, wherein a ratio of the leg opening length to the minimum crotch region width of the fourth disposable incontinence diaper (104) BL4/SB4 has a value of 1.1-1.7, in particular 1.2-1.6, more particularly 1.3-1.5.

16. Arrangement according to one of the preceding claims, wherein the first disposable incontinence diaper (101) and the second disposable incontinence diaper (102) and the third disposable incontinence diaper (103) have a respective maximum width of the back region (21) and a respective maximum width of the front region (22), and wherein the maximum width of the back region (21) differs from the maximum width of the front region (22) of the respective first disposable incontinence diaper (101) and of the second disposable incontinence diaper (102) and of the third disposable incontinence diaper (103) by at most 10%, in particular at most 7%, more particularly at most 5%, wherein more particularly the maximum width of the back region (21) is the same as the maximum width of the front region (22) of a respective first disposable incontinence diaper (101) and of the second disposable incontinence diaper (102) and of the third disposable incontinence diaper (103).

17. Arrangement according to one of the preceding Claims 2 to 16, wherein the fourth disposable incontinence diaper (104) has a maximum width of the back region (21) and a respective maximum width of the front region (22), and wherein the maximum width of the back region (21) differs from the maximum width of the front region (22) of the fourth disposable incontinence diaper (104) by at most 10%, in particular at most 7%, more particularly at most 5%, wherein more particularly the maximum width of the back region (21) is the same as the maximum width of the front region (22) of the fourth disposable incontinence diaper (104).

18. Arrangement according to either of Claims 16 and 17, wherein a first ratio of the maximum width of the back region (21) to the leg opening length of the first disposable incontinence diaper (101) RB1/BL1 is greater than a second ratio of the maximum width of the back region (21) to the leg opening length of the second disposable incontinence diaper (102) RB2/BL2, and wherein the second ratio of the maximum width of the back region (21) to the leg opening length of the second disposable incontinence diaper (102) RB2/BL2 is greater than a third ratio of the maximum width of the back region (21) to the leg opening length of the third disposable incontinence diaper (103) RB3/BL3, such that: RB1/BL1 > RB2/BL2 and RB2/BL2 > RB3/BL3.

19. Arrangement according to one of the preceding claims, wherein the first and the second and the third and/or the fourth disposable incontinence diaper comprises a liquid absorption and distribution layer.

20. Arrangement according to one of the preceding claims, wherein the absorption body (4) of the first (101) and the second (102) and the third (103) and/or the fourth disposable incontinence diaper (104) comprises or consists of a first absorbent ply (4a), and wherein the first absorbent ply (4a) preferably comprises cellulose or cellulose and superabsorbent polymer (SAP).

21. Arrangement according to Claim 20, wherein the absorption body of the first and second and third and/or fourth disposable incontinence diaper comprises a second absorbent ply, and wherein the second absorbent ply is arranged between the first absorbent ply and the backsheet.

22. Arrangement according to either of Claims 20 and 21, wherein the first absorbent ply has at least one channel extending in the longitudinal direction.

## Revendications

1. Agencement comprenant au moins une première couche jetable pour incontinence (101), une deuxième couche jetable pour incontinence (102) et une troisième couche jetable pour incontinence (103), l'agencement comprenant respectivement des emballages contenant au moins un représentant de la première, de la deuxième et de la troisième couche jetable pour incontinence, la première couche jetable pour incontinence (101) présentant une première longueur de châssis CL1, la deuxième couche jetable pour incontinence (102) présentant une deuxième longueur de châssis CL2 et la troisième couche jetable pour incontinence (103) présentant une troisième longueur de châssis CL3,
- la première (101), la deuxième (102) et la troisième couche jetable pour incontinence (103) présentant chacune une direction longitudinale (8) et, orthogonalement à la direction longitudinale (8), une direction transversale (9), et
- la première (101), la deuxième (102) et la troisième couche jetable pour incontinence (103) comprenant chacune une feuille supérieure (2) au moins partiellement perméable aux liquides, une feuille arrière (3) essentiellement imperméable aux liquides et un corps absorbant (4) disposé entre la feuille supérieure (2) et la feuille arrière (3),
- avec CL1>CL2 et CL2>CL3,
- la première couche jetable pour incontinence (101), la deuxième couche jetable pour incontinence (102) et la troisième couche jetable pour incontinence (103) comprenant chacune une partie principale (11) comprenant une région avant (14) et une région arrière (12) ainsi qu'une région d'entrejambe (13) disposée entre celles-ci dans la direction longitudinale (8), destinée à être située entre les jambes d'un utilisateur,
- et la première (101) et la deuxième couche jetable pour incontinence (102) comprenant chacune, de part et d'autre, des sections latérales arrière (17) essentiellement rectangulaires, fixées aux bords longitudinaux latéraux arrière (15) de la région arrière (12) et espacées l'une de l'autre dans la direction transversale (9), qui s'étendent dans la direction transversale (9) au-delà des bords longitudinaux latéraux arrière (15) de la région arrière (12),
- et la première (101) et la deuxième couche jetable pour incontinence (102) comprenant chacune, de part et d'autre, des sections latérales avant (18) essentiellement rectangulaires, fixées aux bords longitudinaux latéraux avant (16) de la région avant (14) et espacées l'une de l'autre dans la direction transversale (9), qui s'étendent dans la direction transversale (9) au-delà des bords longitudinaux latéraux avant (16) de la région avant (14),
- et les sections latérales arrière (17) étant espacées des sections latérales avant (18) dans la direction longitudinale (8),
- la première (101) et la deuxième couche jetable pour incontinence (102) présentant chacune, dans la région d'entrejambe (13), de part et d'autre dans la direction transversale (9), une ouverture de jambe (5),
- chacune des sections latérales arrière (17) présentant respectivement deux moyens de fermeture (19) espacés l'un de l'autre dans la direction longitudinale (8), de sorte que les sections latérales arrière (17) peuvent, pour la mise en place et la fermeture de la première (101) ou de la deuxième couche jetable pour incontinence (102) correspondante sur un utilisateur, être respectivement enroulées autour du corps de l'utilisateur le long d'une direction circonférentielle et amenées dans une disposition de recouvrement avec un côté extérieur de la région avant (14) et/ou un côté extérieur des sections latérales avant (18), auxquelles elles peuvent être fixées de manière amovible respectivement par le moyen de fermeture (19) correspondant,
- la troisième couche jetable pour incontinence (103) présentant, dans la région arrière (12), de part et d'autre dans la direction transversale (9), des oreilles arrière de couche (27) s'étendant vers l'extérieur et, dans la région avant (14), de part et d'autre dans la direction transversale (9), des oreilles avant de couche (28) s'étendant vers l'extérieur, les oreilles arrière de couche (27) et les oreilles avant de couche (28) étant formées en une unité structurelle avec la partie principale (11),
- chacune des deux oreilles arrière de couche (27) présentant respectivement un unique moyen de fermeture (29), de sorte que les oreilles arrière de couche (27) peuvent, pour la mise en place et la fermeture de la troisième couche jetable pour incontinence (103) sur un utilisateur, être respectivement enroulées autour du corps de l'utilisateur le long d'une direction circonférentielle et amenées dans une disposition de recouvrement avec un côté extérieur de la région avant (14) et/ou un côté extérieur des oreilles avant de couche (28), auxquels elles peuvent être fixées de manière amovible respectivement par le moyen de fermeture (29) correspondant,
- et la troisième couche jetable pour incontinence (103) présentant, dans la région d'entrejambe (13), de part et d'autre, une ouverture de jambe profilée (25).

2. Agencement selon la revendication 1 comprenant une quatrième couche jetable pour incontinence (104) présentant une longueur de châssis CL4, une direction longitudinale (8), une direction transversale (9) orthogonale à la direction longitudinale (8), une feuille supérieure (2) au moins partiellement perméable aux liquides, une feuille arrière (3) essentiellement imperméable aux liquides et un corps absorbant (4) disposé entre la feuille supérieure (2) et la feuille arrière (3),
- avec CL3>CL4,
- la quatrième couche jetable pour incontinence (104) comprenant une partie principale (11) comprenant une région avant (14) et une région arrière (12) ainsi qu'une région d'entrejambe (13) disposée entre celles-ci dans la direction longitudinale (8) et destinée à être située entre les jambes d'un utilisateur,
- la quatrième couche jetable pour incontinence (104) présentant, dans la région arrière (12), de part et d'autre dans la direction transversale (9), des oreilles arrière de couche (27) s'étendant vers l'extérieur et, dans la région avant (14), de part et d'autre dans la direction transversale (9), des oreilles avant de couche (28) s'étendant vers l'extérieur, les oreilles arrière de couche (27) et les oreilles avant de couche (28) étant formées en une unité structurelle avec la partie principale (11),
- chacune des deux oreilles arrière de couche (27) présentant respectivement un unique moyen de fermeture (29), de sorte que les oreilles arrière de couche (27) peuvent, pour la mise en place et la fermeture de la quatrième couche jetable pour incontinence (104) sur un utilisateur, être respectivement enroulées autour du corps de l'utilisateur le long d'une direction circonférentielle et amenées dans une disposition de recouvrement avec un côté extérieur de la région avant (14) et/ou un côté extérieur des oreilles avant de couche (28), auxquels elles peuvent être fixées de manière amovible respectivement par le moyen de fermeture (29) correspondant,
- et la quatrième couche jetable pour incontinence (104) présentant, dans la région d'entrejambe (13), de part et d'autre, une ouverture de jambe profilée (25).

3. Agencement selon l'une quelconque des revendications précédentes, dans lequel au moins un des moyens de fermeture (19) de la première (101) et/ou de la deuxième couche jetable pour incontinence (102) présente une première longueur de moyen de fermeture VL1 dans la direction longitudinale (8) de la couche jetable pour incontinence, et dans lequel au moins un des moyens de fermeture (19) de la troisième (103) et/ou de la quatrième couche jetable pour incontinence (104) présente une deuxième longueur de moyen de fermeture VL2 dans la direction longitudinale (8) de la couche jetable pour incontinence, et dans lequel VL1 < VL2.

4. Agencement selon la revendication 3, dans lequel un rapport de VL2 à VL1 présente une valeur de 1,2 à 3,0, notamment 1,3 à 2,7, plus particulièrement 1,5 à 2,5, plus particulièrement 1,8 à 2,2, et plus particulièrement de 2,0.

5. Agencement selon l'une quelconque des revendications précédentes, dans lequel l'ouverture de jambe (5) de la première couche jetable pour incontinence (101) présente une première longueur d'ouverture de jambe BL1, et dans lequel l'ouverture de jambe (5) de la deuxième couche jetable pour incontinence (102) présente une deuxième longueur d'ouverture de jambe BL2, et dans lequel l'ouverture de jambe profilée (25) de la troisième couche jetable pour incontinence (103) présente une troisième longueur d'ouverture de jambe BL3, et dans lequel, notamment, BL1 > BL2 et BL2 > BL3.

6. Agencement selon la revendication 5, dans lequel l'ouverture de jambe profilée (25) de la quatrième couche jetable pour incontinence (104) présente une quatrième longueur d'ouverture de jambe BL4, et dans lequel, notamment, BL3 > BL4.

7. Agencement selon l'une quelconque des revendications 5 ou 6, dans lequel un rapport de la longueur de châssis à la longueur d'ouverture de jambe de la première couche jetable pour incontinence (101) CL1/BL1, de la deuxième couche jetable pour incontinence (102) CL2/BL2 et de la troisième couche jetable pour incontinence (103) CL3/BL3 présente respectivement une valeur de 1,6 à 2,4, notamment de 1,7 à 2,3, plus particulièrement de 1,8 à 2,2, et plus particulièrement de 1,9 à 2,2.

8. Agencement selon l'une quelconque des revendications 6 ou 7, dans lequel un rapport de la longueur de châssis à la longueur d'ouverture de jambe de la quatrième couche jetable pour incontinence (104) CL4/BL4 présente une valeur de 1,6 à 2,4, notamment de 1,7 à 2,3, plus particulièrement de 1,8 à 2,2, et plus particulièrement de 1,9 à 2,2.

9. Agencement selon l'une quelconque des revendications 5 à 8, dans lequel la longueur d'ouverture de jambe de l'ouverture de jambe (5) de la première couche jetable pour incontinence (101) BL1 et de la deuxième couche jetable pour incontinence (102) BL2 présente une valeur de 380 à 490 mm, notamment 390 à 480 mm, plus particulièrement 400 à 470 mm, et plus particulièrement 410 à 460 mm.

10. Agencement selon l'une quelconque des revendications 5 à 9, dans lequel la longueur d'ouverture de jambe de l'ouverture de jambe profilée (25) de la troisième couche jetable pour incontinence (103) BL3 présente une valeur de 280 à 370 mm, notamment de 290 à 360 mm, et plus particulièrement de 300 à 350 mm.

11. Agencement selon l'une quelconque des revendications 6 à 10, dans lequel la longueur d'ouverture de jambe de l'ouverture de jambe profilée (25) de la quatrième couche jetable pour incontinence (104) BL4 présente une valeur de 280 à 370 mm, notamment de 290 à 360 mm, et plus particulièrement de 300 à 350 mm.

12. Agencement selon l'une quelconque des revendications précédentes, dans lequel une largeur minimale de la région d'entrejambe de la première couche jetable pour incontinence (101) SB1 et une largeur minimale de la région d'entrejambe de la deuxième couche jetable pour incontinence (102) SB2 sont respectivement supérieures à une largeur minimale de la région d'entrejambe de la troisième couche jetable pour incontinence (103) SB3.

13. Agencement selon la revendication 12, dans lequel la largeur minimale de la région d'entrejambe de la première couche jetable pour incontinence (101) SB1 et la largeur minimale de la région d'entrejambe de la deuxième couche jetable pour incontinence (102) SB2 sont respectivement supérieures à une largeur minimale de la région d'entrejambe de la quatrième couche jetable pour incontinence (104) SB4, dans lequel, en particulier, SB1 = SB2, et plus particulièrement, SB3 = SB4.

14. Agencement selon l'une quelconque des revendications 12 ou 13, dans lequel un rapport de la longueur d'ouverture de jambe à la largeur minimale de la région d'entrejambe de la première couche jetable pour incontinence (101) BL1/SB1, de la deuxième couche jetable pour incontinence (102) BL2/SB2 et de la troisième couche jetable pour incontinence (103) BL3/SB3 présente une valeur de 1,1 à 1,7, notamment 1,2 à 1,6, et plus particulièrement 1,3 à 1,5.

15. Agencement selon l'une quelconque des revendications 13 ou 14, dans lequel un rapport de la longueur d'ouverture de jambe à la largeur minimale de la région d'entrejambe de la quatrième couche jetable pour incontinence (104) BL4/SB4 présente une valeur de 1,1 à 1,7, notamment 1,2 à 1,6, et plus particulièrement 1,3 à 1,5.

16. Agencement selon l'une quelconque des revendications précédentes, dans lequel la première couche jetable pour incontinence (101), la deuxième couche jetable pour incontinence (102) et la troisième couche jetable pour incontinence (103) présentent chacune une largeur maximale de la région arrière (21) et une largeur maximale de la région avant (22), et dans lequel la largeur maximale de la région arrière (21) diffère de la largeur maximale de la région avant (22) de la première couche jetable pour incontinence (101), de la deuxième couche jetable pour incontinence (102) et de la troisième couche jetable pour incontinence (103) respective d'au plus 10 %, notamment d'au plus 7 %, plus particulièrement d'au plus 5 %, la largeur maximale de la région arrière (21) étant en outre, plus particulièrement, égale à la largeur maximale de la région avant (22) de la première couche jetable pour incontinence (101), de la deuxième couche jetable pour incontinence (102) et de la troisième couche jetable pour incontinence (103) respective.

17. Agencement selon l'une quelconque des revendications 2 à 16, dans lequel la quatrième couche jetable pour incontinence (104) présente une largeur maximale de la région arrière (21) et une largeur maximale de la région avant (22), et dans lequel la largeur maximale de la région arrière (21) diffère de la largeur maximale de la région avant (22) de la quatrième couche jetable pour incontinence (104) d'au plus 10 %, notamment d'au plus 7 %, plus particulièrement d'au plus 5 %, la largeur maximale de la région arrière (21) étant en outre, plus particulièrement, égale à la largeur maximale de la région avant (22) de la quatrième couche jetable pour incontinence (104).

18. Agencement selon l'une quelconque des revendications 16 ou 17, dans lequel un premier rapport de la largeur maximale de la région arrière (21) à la longueur d'ouverture de jambe de la première couche jetable pour incontinence (101) RB1/BL1 est supérieur à un deuxième rapport de la largeur maximale de la région arrière (21) à la longueur d'ouverture de jambe de la deuxième couche jetable pour incontinence (102) RB2/BL2, et dans lequel le deuxième rapport de la largeur maximale de la région arrière (21) à la longueur d'ouverture de jambe de la deuxième couche jetable pour incontinence (102) RB2/BL2 est supérieur à un troisième rapport de la largeur maximale de la région arrière (21) à la longueur d'ouverture de jambe de la troisième couche jetable pour incontinence (103) RB3/BL3, de sorte que : RB1/BL1 > RB2/BL2 et RB2/BL2 > RB3/BL3.

19. Agencement selon l'une quelconque des revendications précédentes, dans lequel la première et la deuxième et la troisième et/ou la quatrième couche jetable pour incontinence comprend une couche d'acquisition et de distribution de liquide.

20. Agencement selon l'une quelconque des revendications précédentes, dans lequel le corps absorbant (4) de la première (101), de la deuxième (102), de la troisième (103) et/ou de la quatrième couche jetable pour incontinence (104) comprend une première couche de corps absorbant (4a) ou consiste en celle-ci, et dans lequel la première couche de corps absorbant (4a) comprend de préférence de la cellulose ou de la cellulose et un polymère superabsorbant (SAP).

21. Agencement selon la revendication 20, dans lequel le corps absorbant de la première et de la deuxième et de la troisième et/ou de la quatrième couche jetable pour incontinence comprend une deuxième couche de corps absorbant, et dans lequel la deuxième couche de corps absorbant est disposée entre la première couche de corps absorbant et la feuille arrière.

22. Agencement selon l'une quelconque des revendications 20 ou 21, dans lequel la première couche de corps absorbant présente au moins un canal s'étendant dans la direction longitudinale.
